# EUROPEAN PATENT APPLICATION

(11) **EP 4 215 614 A1**
(43) Date of publication of application: **26.07.2023**
(21) Application number: 22305070.9
(22) Date of filing: 24.01.2022
(51) Int. Cl.: C12N 15/113

(54) **COMBINATION THERAPY FOR DYSTROPHIN-RELATED DISEASES**

(71) Applicant: Dynacure, 67400 Illkirch-Graffenstaden (FR)
(72) Inventor: Cowling, Belinda, Illkirch-Graffenstaden (FR)
(74) Representative: Cabinet Becker et Associés

(57) **Abstract**

The present invention relates to a new therapy for dystrophin-related diseases. More particularly, the present invention relates to a combination therapy for use in treating a dystrophin-related myopathy in a subject in need thereof, said therapy comprising (i) a dynamin 2 inhibitor and (ii) a therapy providing or restoring a functional dystrophin in the subject.

## Description

### INTRODUCTION

The present invention relates to a new therapy for dystrophin-related diseases. More particularly, the present invention relates to a combination therapy for use in treating a dystrophin-related myopathy in a subject in need thereof, comprising (i) a dynamin 2 inhibitor and (ii) a therapy providing or restoring a functional dystrophin in the subject.

The dystrophin *DMD* gene is the longest human gene with 2.4 megabases of DNA accounting for about 1% of the chromosome X DNA. It is localized on the locus p21 of chromosome X and codes for the dystrophin protein. This gene comprises 79 exons separated by very large introns which explain the giant size of the gene; the mRNA of the largest isoform of dystrophin is 14 kb (i.e. only 0.6% of the total weight of the gene). The full-length dystrophin is predominantly expressed in all striated (skeletal, cardiac) and smooth muscles, while shorter isoforms are expressed in small amounts in the brain and retina. In muscles, the dystrophin protein forms the mechanical link between the contractile apparatus and the plasma membrane of the muscle fiber. It consists of four major structural domains: (1) the N-terminal domain coded by exons 1 to 8, which is mostly an actin-binding domain, (2) the central rod domain coded by exons 9 to 61, which interacts with numerous proteins (filamentous actin, intermediate filaments, microtubules, etc.) and membrane phospholipids, (3) the cysteine-rich domain coded by exons 62 to 70, which binds, *inter alia,* to the membrane protein beta-dystroglycan (this domain is thus named the dystroglycan binding site), and (4) the C-terminal domain coded by exons 71 to 79, which binds to cytoplasmic proteins. Thanks to this structure, the dystrophin protein can anchor to plasma membrane and interact with extracellular matrix proteins. It forms a scaffolding network at specific muscle structures named costameres, that are involved in the lateral transmission of forces which allow muscle fibers to contract, whilst maintaining membrane integrity and preventing major plasma membrane ruptures. Disruption to proteins in this complex can result in reduced muscle contraction, and/or damage to plasma membranes which may result in the eventual destruction of muscle fibers.

Mutations in the dystrophin *DMD* gene are the cause of two devastating diseases, namely Duchenne (DMD) and Becker (BMD) muscular dystrophies. Depending upon the preservation or not of the reading frame, dystrophin is completely absent in DMD, or present in either a mutated or a truncated form in BMD. These defects result in an impairment of the transmission of forces from the cytosol to the extracellular matrix at the costameres, which leads to frequent ruptures of plasma membrane during contractions and in turn to the leakage of cellular components such as creatine kinase and to the influx of calcium. In other words, these mutations generate a large amount of stress on the muscle fibers, which will become damaged because these are not able to tolerate repetitive forces over time.

Due to the total loss of dystrophin, the first clinical signs of Duchenne muscular dystrophy (DMD) are severe and appear at an early age, generally around 2-5 years of age. Children affected by DMD, which are mostly boys, typically encounter difficulties first to walk and climb, then to run and breath, and will become wheelchair bound by 12-16 years old of age. These clinical signs, also accompanied by elevated creatine kinase levels, are due to a progressive muscle wasting and weakness affecting almost all muscles, which will ultimately result in a premature death from cardiorespiratory failure. Histologically, DMD muscles are characterized by alternating cycles of fiber necrosis and regeneration. Over time, the ongoing regeneration process is exhausted, and fibrosis and adipose tissue replacement of muscle fibers occurs. Lesions of the plasma membrane can be revealed by electron microscopy, and the absence of dystrophin can be observed by immunoblotting or fluorescent labelling, except in some revertant fibers.

Becker muscular dystrophy (BMD) is characterized by similar clinical signs, albeit with a very variable time course and severity. Some BMD patients are relatively asymptomatic, while others may become be wheelchair-dependent early on. This means that some will survive until an advanced age, while others will have a premature death. The expression of mutated dystrophin can be observed in BMD muscles, with a high variable extent ranging from less than 10% to 70% of the full-length expression of healthy muscles.

Effective therapies are required for DMD and BMD patients which target the physiological muscle force, and therefore improve muscle function. To this day, despite the great progress made in research into these therapies and their ongoing development (most targeting dystrophin expression), no transformative therapy has yet been clinically approved for these diseases (Al Zaidi et al., Pediatr Neurol 2014; 51(5):607-618).

The present Inventors are herein the first to investigate an innovative approach aimed at combining (i) a reduction in DNM2 expression, activity or function with (ii) a restoration of a functional dystrophin, so as to prevent or delay the onset and/or disease progression. To do so, they assessed the efficacy of this proof-of-concept in an animal model of dystrophin-related myopathy, using exon-skipping in the *Dmd* gene in combination with a knockdown of the *Dnm2* mRNA - both via antisense oligonucleotides. This combined therapy surprisingly produced a synergistic improvement in muscle function, thereby confirming the suitability of this approach for treating dystrophin-related myopathies. Such combination is also advantageous in that it can allows a reduction in amount of each therapeutic agent, while still achieving a therapeutic effect. It should nevertheless be understood that the combination of (i) any dynamin 2 inhibitor with (ii) any therapy providing or restoring a functional dystrophin would achieve the same effect.

### SUMMARY OF THE INVENTION

In a first aspect, the invention relates to a combination therapy for use in the treatment of a dystrophin-related myopathy in a subject in need thereof, the combination therapy comprising (i) a dynamin 2 inhibitor and (ii) a therapy providing or restoring a functional dystrophin in the subj ect.

In a preferred embodiment, the dystrophin-related myopathy is Duchenne muscular dystrophy or Becker muscular dystrophy, preferably Duchenne muscular dystrophy.

In a preferred embodiment, the dynamin 2 inhibitor is selected from the group consisting of a nucleic acid molecule interfering specifically with dynamin 2 expression, an antibody directed against dynamin 2, a genome editing system comprising a nuclease engineered to target the *DNM2* gene, and a small molecule inhibiting the dynamin 2 activity, expression or function, and any combinations thereof.

In a preferred embodiment, the nucleic acid molecule interfering specifically with dynamin 2 expression is an antisense nucleic acid, an RNAi nucleic acid, or a ribozyme interfering specifically with dynamin 2 expression.

In a preferred embodiment, the nucleic acid molecule interfering specifically with dynamin 2 expression is complementary to at least one intron or the 3'UTR of dynamin 2 pre-mRNA, preferably to at least intron 12, intron 13, intron 11, intron 1, intron 14 of dynamin 2 pre-mRNA, more preferably is an antisense nucleic acid comprising or consisting of at least 8 contiguous nucleobases of any one of SEQ ID NO: 1 to 3135.

In a preferred embodiment, the nucleic acid molecule interfering specifically with dynamin 2 expression induces exon-skipping within a dynamin 2 pre-mRNA, preferably of at least exon 2 and/or 8 of dynamin 2 pre-mRNA, more preferably is an antisense nucleic acid comprising or consisting of the sequence SEQ ID NO: 3136 or SEQ ID NO: 3137.

In a preferred embodiment, the nucleic acid molecule interfering specifically with dynamin 2 expression is complementary to at least one exon of dynamin 2 mRNA, preferably to at least exon 1, 4, 5, 12b, 13, 15, 17 and/or 21 of dynamin 2 mRNA, more preferably is an RNAi nucleic acid comprising or consisting of any one of the sequences SEQ ID NO: 3138 to SEQ ID NO: 3151.

In a preferred embodiment, the product providing the subject with a functional dystrophin is selected from the group consisting of: dystrophin gene therapy product, exon-skipping therapy, codon-read-through therapy, genome editing therapy, myogenic cell therapy, and any combinations thereof.

In a preferred embodiment, the dystrophin gene therapy provides a functional dystrophin selected from a mini- or micro-dystrophin or a full-length dystrophin , preferably selected from the micro-dystrophins ΔDysM3, ΔDysH1, ΔDysH4, ΔDysHAH3, ΔDysAX2, ΔDysAX11, Δ3447, Δ3510, Δ3531, Δ3849, Δ3990, Δ4173, ΔR1-R24, ΔR4-23/ΔCT, ΔR4-R23, ΔR2-R21, AR2-R21+H3, Δ3788, ABS1µDys, ABS1.2µDys, ΔR2-15/ΔR18-23/ΔC, ΔR3-15/ΔR18-23/AC, ΔR3-15/ΔR17-23/ΔC, M1, M2, M3, M4, ΔH2-R24/ΔCT, ΔH2-R23+H3/ΔCT, ΔH2-R23+R18-H3/ΔCT, ΔR4-R23/ΔCT/Δ, MD1, MD2, ΔCS2, R6-17/H3/ΔC, or mDys5, or the mini-dystrophin Δ17-48; or any combinations thereof.

In a preferred embodiment, the exon-skipping therapy induces exon-skipping of at least one mutated exon of a dystrophin pre-mRNA, preferably of at least one of exon 51, 53, 45, 44, 52, 43, 23, 35, 50, 52, 7, 8, 17 and/or 55 of a dystrophin pre-mRNA, more preferably comprises an exon-skipping antisense nucleic acid such as eteplirsen, drisapersen, SRP50-51, suvodirsen, golodirsen, viltolarsen, WVE-N531, casimersen, DS-5141b, NS-89/NCNP-02, or any combinations thereof.

In a preferred embodiment, the codon-read-through therapy comprises a small molecule suppressing premature termination codons in a dystrophin mRNA, said small molecule being preferably selected from the group consisting of aminoglycoside compounds such as geneticin, gentamycin, gentamycin B1, paromomycin, amikacin, NB30, NB54, NB74, NB84, NB124, TC007; and non-aminoglycoside compounds such as ataluren, PCT-414, RTC13, RTC14, GJ071, GJ072, RTC204, RTC219, BZ6, BZ16, negamycin, 3-epideoxynegamycin, leucyl-3-epi-deoxynegamycin, TCP-112, TCP-182, TCP-1109, amlexanox, clitocine, 2,6-diaminopurine, tylosin, j osamycin, spiramycin, erythromycin, azithromycin; functional analogs thereof, and any combinations thereof.

In a preferred embodiment, the myogenic cellular therapy comprises precursor cells, progenitor cells, or stem cells that are capable of displaying a myogenic phenotype, preferably comprises myoblasts, satellite cells, mesoangioblasts, muscle-side population cells, cardiosphere-derived cells, induced pluripotent stem cells (iPSC), or any combinations thereof.

In a preferred embodiment, the dynamin 2 inhibitor is administered in an amount sufficient to reduce the dynamin 2 expression, activity, expression or function in a level equal or preferably less than the normal level; and/or the therapy providing or restoring a functional dystrophin is administered in an amount sufficient to restore at least 1%, 5%, 10%, or 15%of expression or function of dystrophin.

In a further aspect, the invention relates to (i) a dynamin 2 inhibitor and (ii) a therapy providing or restoring a functional dystrophin, as described herein, as a combined preparation for simultaneous, separate or sequential use in the treatment of a dystrophin-related myopathy, such as Duchenne muscular dystrophy or Becker muscular dystrophy.

In another aspect, the invention relates to a pharmaceutical composition comprising (i) a dynamin 2 inhibitor and (ii) a therapy providing or restoring a functional dystrophin, as described herein, and optionally a pharmaceutically acceptable excipient.

### LEGENDS TO THE FIGURES

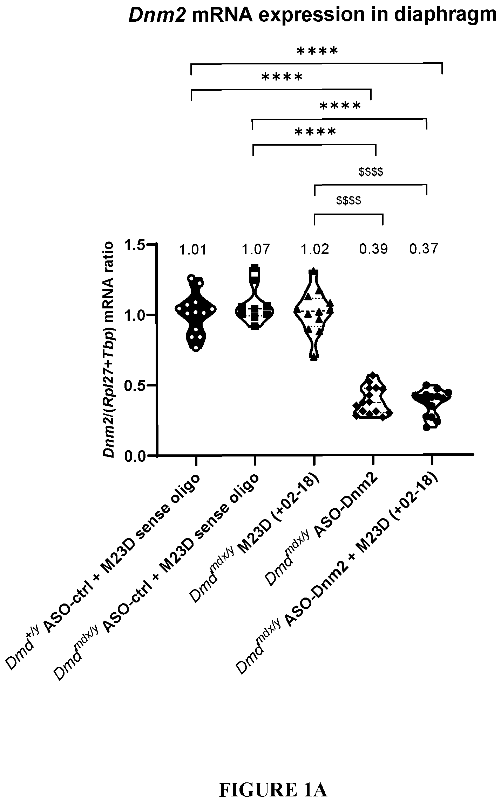

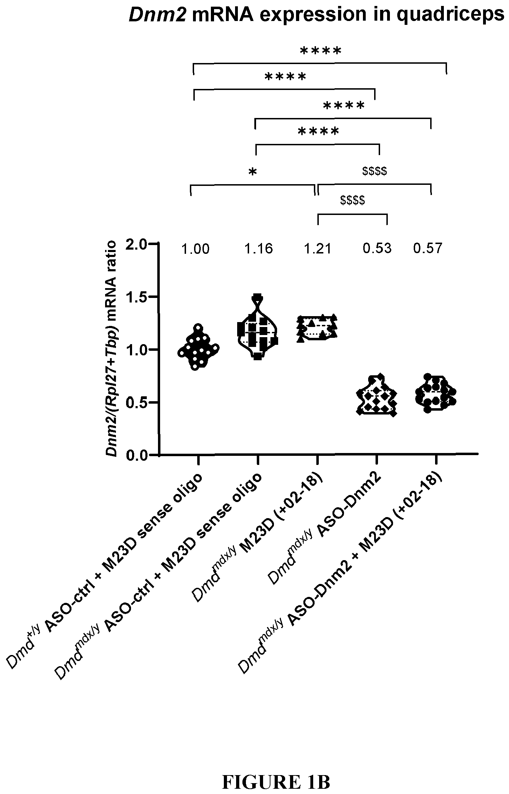

**Figure 1****. Analysis of *Dnm2* mRNA levels following ASO administration in mice.** *Dnm2* mRNA levels were measured relative to *Rpl27*+*Tbp* mRNA levels in 2 skeletal muscles, diaphragm **(A)** and quadriceps **(B),** of 20 weeks-old DBA/2J wildtype mice treated with control ASOs (*Dmd*^{+/*y*} ASO-ctrl + M23D sense oligo), *mdx* mice treated with control ASOs (*Dmd*^{*mdx*/*y*} ASO-ctrl + M23D sense oligo), *mdx* mice treated with an exon-skipping ASO (*Dmd*^{*mdx*/*y*} M23D (+02-18)), *mdx* mice treated with an ASO against *Dnm2* (*Dmd*^{*mdx*/*y*} ASO-Dnm2) and *mdx* mice treated with both ASOs (*Dmd*^{*mdx*/*y*} ASO-Dnm2 + M23D (+02-18)). Violin plots are represented. A one-way ANOVA test followed by a Sidak's multiple comparisons *post-hoc* test was performed, p-values: ^{∗∗∗∗} < 0.0001 (comparisons with both *Dmd*^{+/y} and *Dmd*^{*mdx*/*y*} control groups); ^{$$$$} < 0.0001 (comparisons between the three treatment groups).

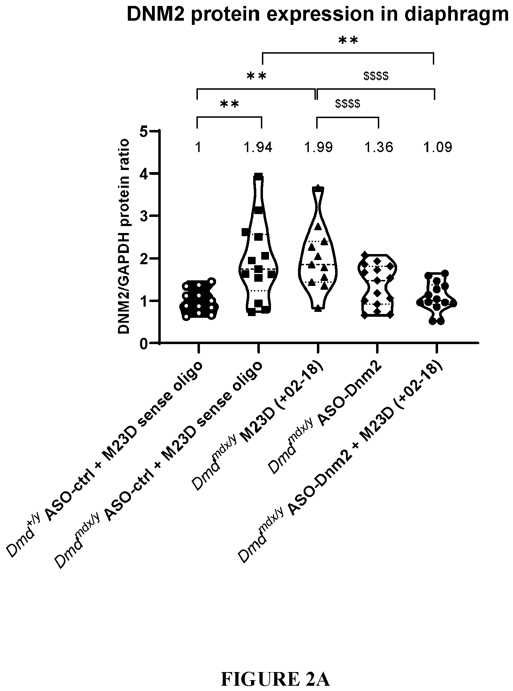

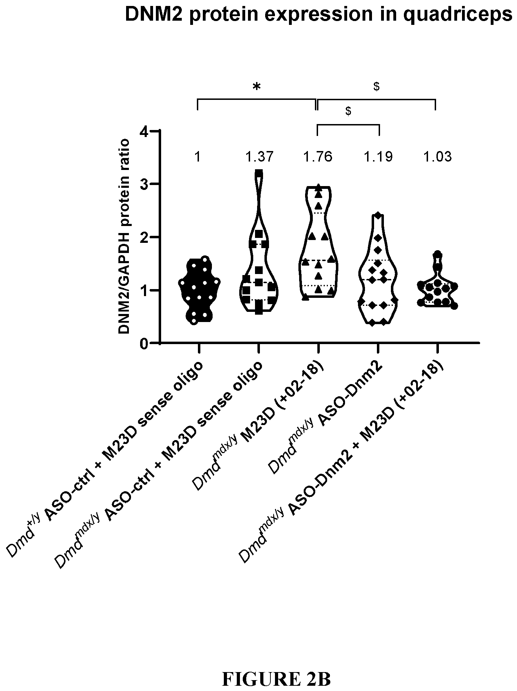

**Figure 2****. Analysis of DNM2 protein levels following ASO administration in mice.** DNM2 protein levels were measured relative to GAPDH protein levels in 2 skeletal muscles, diaphragm **(A)** and quadriceps **(B),** of 20 weeks-old DBA/2J wildtype mice treated with control ASOs (*Dmd*^{+/*y*} ASO-ctrl + M23D sense oligo), *mdx* mice treated with control ASOs (*Dmd*^{*mdx*/*y*} ASO-ctrl + M23D sense oligo), *mdx* mice treated with an exon-skipping ASO (*Dmd*^{*mdx*/*y*} M23D (+02-18)), *mdx* mice treated with an ASO against *Dnm2* (*Dmd*^{*mdx*/*y*} ASO-Dnm2) and *mdx* mice treated with both ASOs (*Dmd*^{*mdx*/*y*} ASO-Dnm2 + M23D (+02-18)). Violin plots are represented. A one-way ANOVA test followed by a Sidak's multiple comparisons *post-hoc* test was performed, p-values: ^{∗} < 0.05; ^{∗∗∗∗} < 0.0001 (comparisons with both *Dmd*^{+/y} and *Dmd*^{*mdx*/*y*} control groups); ^{$$$$} < 0.0001 (comparisons between the three treatment groups).

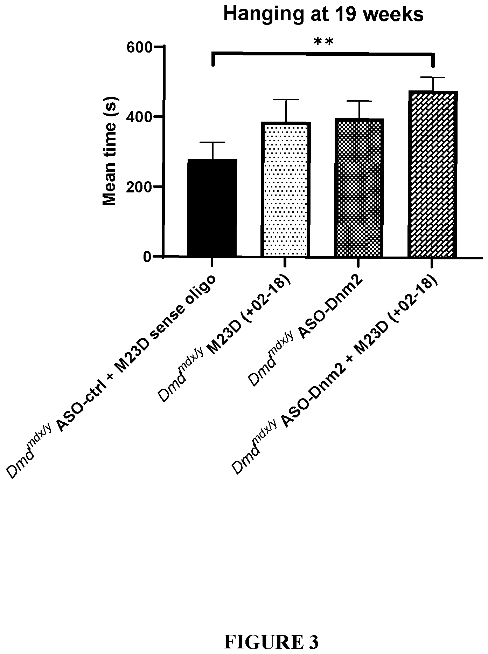

**Figure 3****. Muscle strength following ASO administration in mice (whole-body strength measured with the 10-minute hanging test).** The test was performed in 19 weeks old DBA/2J *mdx* mice treated with control ASOs (*Dmd*^{*mdx*/*y*} ASO-ctrl + M23D sense oligo), *mdx* mice treated with an exon-skipping ASO (*Dmd*^{*mdx*/*y*} M23D (+02-18)), *mdx* mice treated with an ASO against *Dnm2* (*Dmd*^{*mdx*/*y*} ASO-Dnm2) and *mdx* mice treated with both ASOs (*Dmd*^{*mdx*/*y*} ASO-Dnm2 + M23D (+02-18)), following 12 weeks of treatment. Mean ± SEM is represented. A non-parametric Kruskal-Wallis test followed by a Dunn's multiple comparisons *post-hoc* test was performed, p-values: ^{∗∗} < 0.01 (comparisons with *Dmd*^{*mdx*/*y*} control group).

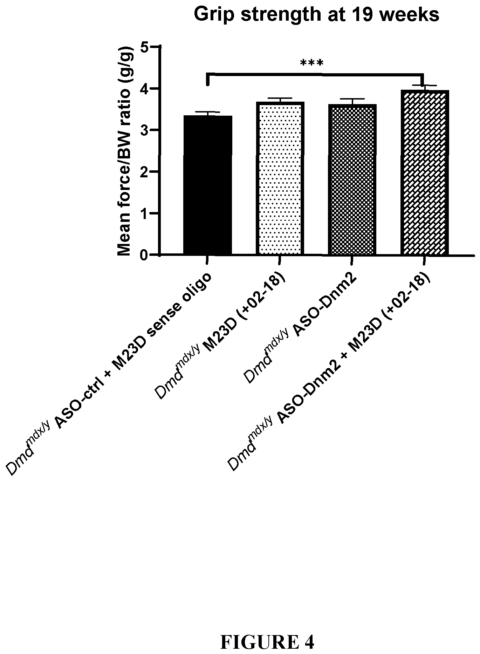

**Figure 4****. Muscle strength following ASO administration in mice (muscle force measured with the grip strength test).** The test was performed in 19 weeks old DBA/2J *mdx* mice treated with control ASOs (*Dmd*^{*mdx*/*y*} ASO-ctrl + M23D sense oligo), *mdx* mice treated with an exon-skipping ASO (*Dmd*^{*mdx*/*y*} M23D (+02-18)), *mdx* mice treated with an ASO against *Dnm2* (*Dmd*^{*mdx*/*y*} ASO-Dnm2) and *mdx* mice treated with both ASOs (*Dmd*^{*mdx*/*y*} ASO-Dnm2 + M23D (+02-18)), following 12 weeks of treatment. Mean ± SEM is represented. A one-way ANOVA test followed by a Dunnett's multiple comparisons *post-hoc* test was performed, p-values: ^{∗∗∗} < 0.001 (comparisons with *Dmd*^{*mdx*/*y*} control group).

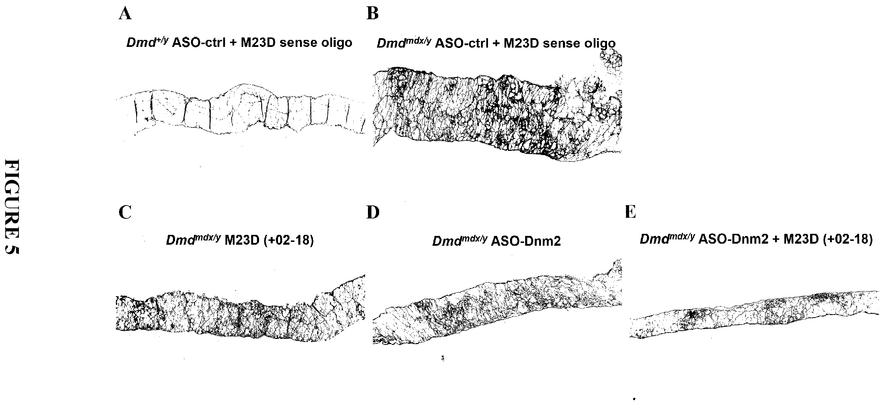

**Figure 5****. Representative images of the diaphragm skeletal muscle following ASO administration in mice.** Images of diaphragm sections from 20 weeks-old DBA/2J wildtype mice treated with control ASOs **(A:** *Dmd*^{+/y} ASO-ctrl + M23D sense oligo), *mdx* mice treated with control ASOs **(B:** *Dmd*^{*mdx*/*y*} ASO-ctrl + M23D sense oligo), *mdx* mice treated with an exon-skipping ASO **(C:** *Dmd*^{*mdx*/*y*} M23D (+02-18)), *mdx* mice treated with an ASO against *Dnm2* **(D:** *Dmd*^{*mdx*/*y*} ASO-Dnm2) and *mdx* mice treated with both ASOs (E: *Dmd*^{*mdx*/*y*} ASO-Dnm2 + M23D (+02-18)) stained with Sirius Red.

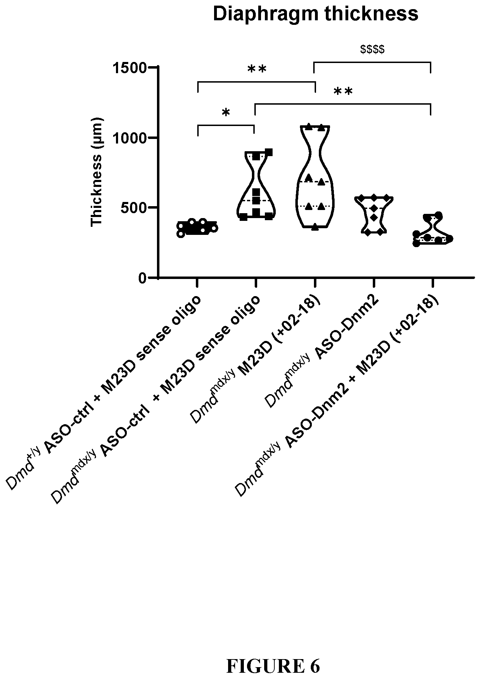

**Figure 6****. Diaphragm thickness following ASO administration in mice.** Diaphragm thickness was measured on histological sections from 20 weeks-old DBA/2J wildtype mice treated with control ASOs (*Dmd*^{+/*y*} ASO-ctrl + M23D sense oligo), *mdx* mice treated with control ASOs (*Dmd*^{*mdx*/*y*} ASO-ctrl + M23D sense oligo), *mdx* mice treated with an exon-skipping ASO (*Dmd*^{*mdx*/*y*} M23D (+02-18)), *mdx* mice treated with an ASO against *Dnm2* (*Dmd*^{*mdx*/*y*} ASO-Dnm2) and *mdx* mice treated with both ASOs (*Dmd*^{*mdx*/*y*} ASO-Dnm2 + M23D (+02-18)). Violin plots are represented. A one-way ANOVA test followed by a Sidak's multiple comparisons *post-hoc* test was performed, p-values: ^{∗} < 0.05; ^{∗∗} < 0.01 (comparisons with both *Dmd*^{+/y} and *Dmd*^{*mdx*/*y*} control groups); ^{$$$$} < 0.0001 (comparisons between the three treatment groups).

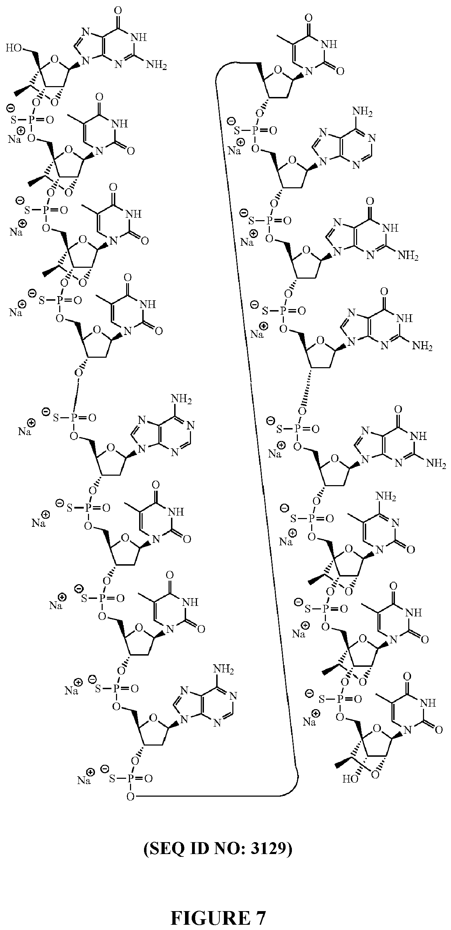

**Figure 7****. Example of a preferred Dynamin 2 inhibitor according to the invention.**

### DETAILED DESCRIPTION OF THE INVENTION

Unless otherwise defined herein, scientific and technical terms used in connection with the present invention shall have the meanings that are commonly understood by those of ordinary skill in the art. Further, unless otherwise required by context, nomenclatures used herein, and techniques of molecular and cellular therapy are those well-known and commonly used in the art.

The present invention may be understood more readily by reference to the following detailed description, included preferred embodiments of the invention, and examples included herein.

The present invention aims at providing an innovative therapeutic approach for the treatment of a dystrophin-related myopathy in a subject in need thereof by (i) reducing DNM2 expression, activity or function and (ii) restoring the dystrophin function in said subject.

Accordingly, in a first aspect, the present invention relates to a combination therapy for use in the treatment of a dystrophin-related myopathy in a subject in need thereof, the combination therapy comprising (i) a dynamin 2 inhibitor and (ii) a therapy providing or restoring a functional dystrophin in the subject

In particular, the present invention is directed to the combined use of (i) a dynamin 2 inhibitor and (ii) a therapy providing or restoring a functional dystrophin, for the preparation of a medicament for the treatment of a dystrophin-related myopathy in a subject in need thereof.

Further provided is a method for treating a dystrophin-related myopathy in a subject in need thereof, said method comprising the administration, to said subject, of a therapeutically effective amount of (i) a dynamin 2 inhibitor and (ii) a therapy providing or restoring a functional dystrophin in the subject.

As used herein, the terms "*dystrophin-related myopathy", "muscular dystrophin-related disease", "muscular dystrophinopathy*" or *"dystrophinopathy*" are interchangeable, and refer to a pathology characterized by muscular dystrophy and which is associated with a defect in muscle dystrophin. Said defect is typically caused by one or more mutations in the dystrophin gene; said pathology can accordingly be referred as a genetic muscular dystrophy. Duchenne (DMD) and Becker (BMD) muscular dystrophies are genetic muscular dystrophies with a relatively high incidence, which are caused by at least one mutation in the dystrophin gene. Phenotypic features of said pathologies are as described above. In DMD, dystrophin is either absent or truncated non-functional, generally as a result of a frameshift mutation in the *DMD* gene, such as a frameshift mutation/out-of-frame deletion or duplication, or a nonsense mutation. By contrast, and as a general rule (i.e. in the majority of patients), BMD is typically caused by a mutation that does not cause a frameshift in the *DMD* gene, thereby generating a partially functional dystrophin; these mutations may be in-frame deletions or duplications of one or more exons, or splice-site mutations. Nevertheless, nonsense and frameshifts mutations leading to some dystrophin with remaining activity have been identified in BMD. Mutations responsible for DMD and BMD have been extensively reviewed in the literature (Muntoni et al., Lancet Neurol, 2003, 2: 731-740; Tuffery-Giraud et al., Hum Mutat., 2009, 30(6):934-45Bladen et al., Hum Mutat. 2015;36(4):395-402;).

Duchenne muscular dystrophy and Becker muscular dystrophy are particularly preferred dystrophin-related myopathies to be treated according to the invention. More preferably, the dystrophin-related myopathy is Duchenne muscular dystrophy.

Generally speaking, the term *"treatment"* or *"treating"* means obtaining a desired physiological or pharmacological effect depending on the degree of severity of the symptom or disorder of interest, or risks thereof, i.e. herein, depending on the degree of severity or risks of developing such symptom or disorder. The effect may be prophylactic in terms of a partial or complete prevention of the symptom or disorder and/or may be therapeutic in terms of a partial or complete cure of the symptom or disorder. The term *"prophylactic"* characterizes the capacity to avoid, or minimize the onset or development of a symptom or disorder before its onset (for example, before the onset of muscle weakness). The term *"therapeutic"* refers to the capacity to inhibit the symptom or disorder (i.e. arresting the development thereof), and/or to relieve said symptom or disorder (i.e. regression leading to an improvement). A prophylactic effect is generally said to be achieved when e.g. an asymptomatic subject remains asymptomatic or quasi-asymptomatic after treatment according the invention (for example, absence of muscle weakness and/or respiratory difficulties), while a therapeutic effect is typically said to be achieved when e.g. a symptomatic subject recovers, partially or totally, after treatment according to the invention (for example, partial or complete recovery of muscle strength and/or respiratory function).

In the context of the present invention, the effectiveness of a treatment may be determined by conducting assays on a myogenic cell or muscle cell from the patient, such as by evaluating reduced calcium uptake by muscle cells, decreased collagen synthesis, altered morphology, altered lipid biosynthesis, decreased oxidative stress, and/or improved muscle fiber function, integrity, and/or survival. Methods for assessing these parameters are well-known in the art. For example, improvement in muscle fiber function, integrity, and/or survival can be determined by using at least one of the following assays: a decrease of necrosis in muscle fibers from a biopsy cross-section of a muscle suspected to be dystrophic, and/or an increase in the homogeneity of the diameter of muscle fibers in a biopsy cross-section of a muscle suspected to be dystrophic. As another example, to evaluate improvement in respiratory function, one can assess structure and function of the diaphragm via Magnetic Resonance Imaging. Alternatively, or additionally, one may use a rating scale such as the one from North Star Ambulatory Assessment (NSAA), to directly measure functional motor abilities of the treated subject, such as prolongation of time to loss of walking, improvement of muscle strength, improvement of the ability to lift weight, improvement of the time taken to rise from the floor, improvement in the nine-meter walking time or 6 minute walk, improvement in the time taken for four-stairs climbing, improvement of the leg function grade, improvement of the pulmonary function, improvement of cardiac function, improvement of the quality of life.

It should be understood that even a partial or intermittent relief of at least one symptom of the dystrophin-related myopathy can be of great benefit for the patient, and is thus considered considered herein as an effective treatment. In addition, treatment of the patient may be a single event, or the patient is administered with the combination therapy on multiple occasions, that may be, depending on the results obtained, several days apart, several weeks apart, or several months apart, or even several years apart. A treatment according to the invention may have a duration of at least one week, at least one month, at least several months, at least one year, at least 2, 3, 4, 5, 6 years or more. The frequency of administration may range between at least once every week, two weeks, three weeks, four weeks or five weeks or a longer time period.

A *"therapeutically effective amount"* means herein an amount that is sufficient to achieve the effect for which it is indicated, herein the treatment of a dystrophin-related myopathy. The amount of the combination therapy to be administered can be determined by standard procedures well known by those of ordinary skill in the art. Physiological data of the patient (e.g. age, size, and weight), the routes of administration and the disease to be treated have to be taken into account to determine the appropriate dosage, optionally compared with subjects that do not suffer from a dystrophin-related myopathy. The amount may also vary according to other components of a treatment protocol (e.g. administration of other medicaments, etc.).

It should be further understood that the *"subject"* or *"host"* to be treated according to the invention is one having a muscular system. Accordingly, preferred subjects to be treated are animals, more preferably mammals, most preferably humans.

The term *"dynamin 2"* (DNM2), also known as CMTDI1, CMTDIB, DI-CMTB, DYN2, dynamin II, or DYNII, refers to a GTPase protein involved in endocytosis, membrane trafficking and fission, and cytoskeleton organization. The dynamin 2 protein contain 5 domains: an N-terminal GTPase domain (G-domain) that binds and hydrolyzes GTP; a middle domain enabling dimerization of the protein; a pleckstrin homology (PH) domain promoting binding to phosphoinositides; a GTPase effector domain (GED) and a C-terminal proline-rich domain (PRD) involved in protein-protein interactions. In humans, dynamin 2 is encoded by *the DNM2* gene located on chromosome 19 and composed of 22 exons (genomic DNA sequence available on the NCBI database under the reference sequence NG_008792.1). The human DNM2 protein exists in five alternatively spliced variants: DNM2 isoform 1 (amino acid sequence: NP_001005360.1, coded by mRNA sequence: NM_001005360.3); DNM2 isoform 2 (amino acid sequence: NP_001005361.1, coded by mRNA sequence: NM_001005361.3); DNM2 isoform 3 (amino acid sequence: NP_004936.2, coded by RNA sequence: NM_004945.4); DNM2 isoform 4 (amino acid sequence: NP_001005362.1, coded by RNA sequence : NM_001005362.3); and DNM2 isoform 5 (amino acid sequence: NP_001177645.1, coded by RNA sequence : NM_001190716.2).

As used herein, a *"dynamin-2 inhibitor"* refers to any molecule capable of decreasing specifically the expression of dynamin 2 or inhibit the dynamin 2 activity or function, either directly or indirectly. Preferably, the dynamin 2 inhibitor is a direct inhibitor, meaning that it interacts directly with either the dynamin 2 protein or a nucleic acid encoding said dynamin 2 or a part thereof. The dynamin 2 inhibitors according to the invention are capable of inhibiting or decreasing the functional activity of dynamin 2 *in vivo* and/or *in vitro.* The inhibitor may inhibit the functional activity of dynamin 2 by at least about 10%, 20%, preferably by at least about 30%, 40%, preferably by at least about 50%, 60%, preferably by at least about 70% or 80%. In particular, the inhibitor may inhibit dynamin 2 expression by at least about 10%, 15%, preferably by at least about 20%, 25%, preferably by at least about 30%, 35%, preferably by at least about 40%, 45%, preferably by at least about 50%, 55%, preferably by at least about 60%, 65%. More particularly, the inhibitor may inhibit dynamin 2 expression by at least about 15%, preferably by at least about 20%, 25%, preferably by at least about 30%, 35%, preferably by at least about 40%, 45%, preferably by at least about 50%, 55%, preferably by at least about 60%.

A dynamin 2 inhibitor of the invention may act by blocking and/or inhibiting the activity or function of dynamin 2. This may for example be achieved by inhibiting the enzymatic activity of dynamin 2. Functional or enzymatic activity of dynamin 2 may be readily assessed by one skilled in the art according to known methods by testing, for example, the GTPase activity or the function of dynamin 2 in clathrin-mediated endocytosis (Macia E. et al, Developmental cell 2006; 10, 839-850). For inhibitors of GTPase activity or lipid binding, subcellular localization, clathrin mediated endocytosis, synaptic vesicle endocytosis, one can use the method described in McCluskey et al. (Traffic, 2013;14(12):1272-89), or McGeachie et al. (ACS Chem Biol, 2013 ;8(7):1507-18). For dynamin 2 GTPase activity, oligomerisation, lipid binding, one can use the method described by Wang et al. (J Biol Chem, 2010; 285(30):22753-7), or by Kenniston and Lemmon (EMBO J, 2010; 29(18):3054-67).

The dynamin 2 inhibitor of the invention may also act by blocking and/or inhibiting the dynamin 2 expression (including transcription, splicing, transcript maturation, or translation). The decrease or inhibition of dynamin 2 expression can be evaluated by any means known to those skilled in the art including, but not limited to, assessing the level of Dynamin 2 protein using for instance Western Blot or ELISA with a specific anti-Dynamin 2 antibody, and/or assessing the level of mRNA transcript for Dynamin 2 using e.g. quantitative PCR.

Dynamin 2 inhibitors have been reported in the scientific and patent literature, and are thus well-known in the art (WO2015055859; WO2018/00010; WO2018189208; WO2019140452; WO2020028844; Tasfaout et al., Nat Commun 2017, 8: 15661; Buono et al., Proc Natl Acad Sci USA 2018, 115(43): 11066-11071; the contents of which are incorporated herein by reference in their entireties). Below is provided a non-exhaustive list of such inhibitors which can be used in the present invention.

The dynamin 2 inhibitor used in the present invention is preferably selected from the group consisting of a nucleic acid molecule interfering specifically with dynamin 2 expression, an antibody directed against dynamin 2, a genome editing system comprising a nuclease and at least one *DNM2* guide RNA, a small molecule inhibiting the dynamin 2 enzymatic activity, expression, or function, and any combinations thereof.

In a preferred embodiment, the dynamin 2 inhibitor used in the present invention is a nucleic acid molecule interfering specifically with dynamin 2 expression.

As used throughout the specification, the terms *"nucleic acid molecule", "nucleic acid*", *"polynucleotide"* (polynucleoside) or *"oligonucleotide"* (oligonucleoside) refers to a succession of natural or synthetic nucleotides (or nucleosides) connected by internucleotide (or internucleoside) linkages, wherein each nucleotide (or nucleoside) or internucleotide (or internucleoside) linkages may be modified or unmodified. A nucleotide is comprised of a nucleoside and a phosphate group. A nucleoside is comprised of a nucleobase and a sugar. A nucleobase comprises a modified and unmodified nucleobase. Unmodified nucleobases are well-known in the art and include adenine (A), thymine (T), cytosine (C), uracil (U) and guanine (G). An internucleotide (internucleoside) linkage is a bond that forms a covalent linkage between adjacent nucleotides (nucleosides) in a nucleic acid, such as phosphate internucleotide linkages that are naturally occurring. A nucleic acid may be a single-stranded or double-stranded DNA such as cDNA, genomic DNA, ribosomal DNA, and the transcription product of said DNA, such as RNA. Nucleic acids also encompass nucleic acids encoding a peptide or polypeptide of interest, as well as nucleic acids which can hybridize to a nucleic acid of reference. When a nucleic acid is designed to hybridize to a nucleic acid of reference, it can be chemically modified to enhance its stability, nuclease resistance, target specificity and/or improve their pharmacological properties (e.g. reduced toxicity, increased intracellular transport, etc.). For example, a nucleic acid may comprise modified nucleotide(s) and/or backbone, such as a modified sugar, a modified nucleobase, and/or a modified internucleotide (internucleoside) linkage. A standard modification is the replacement of native phosphates of the internucleotide (internucleoside) linkage with phosphorothioates (PS) so as to reduce the sensitivity to nucleases and accordingly improve the stability, half-life and tissue distribution of the nucleic acid (Eckstein F., Antisense and Nucleic Acid Drug Development, 2000, 10(2): 1117-221). Another standard modification which increases the affinity of the nucleic acid towards its target is the incorporation in the sugar moiety of a methoxyethyl (MOE) or a constrained ethyl (cEt) to the 2'position, or the tethering of the 4'-carbon to the 2-hydroxyl of the ribose ring to create a bicyclic locked nucleic acid (LNA), to name a few.

Preferred nucleic acid molecules interfering specifically with dynamin 2 expression according to the invention are those capable of hybridizing specifically to the gene or transcripts encoding dynamin 2, at least to a part thereof; as such, these are usually non-naturally occurring nucleic acids (i.e. synthetic).

"*Hybridizing*" or *"hydridization"* means the pairing or annealing to a target, herein under physiological conditions, typically via hydrogen bonding between complementary nucleotides, such as Watson-Crick, Hoogsteen or reversed Hoogsteen hydrogen bonding. A specific hybridization therefore means that the pairing or annealing is specific to the target (no off-targets effects, or at least no substantial off-targets effects).

*"A nucleic acid molecule capable of hybridizing"* to a target nucleic acid thus means that a stretch of this nucleic acid is capable of forming base pairs to another stretch of the target nucleic acid. It is thus not absolutely required that all the bases in the region of complementarity are capable of pairing with bases in the opposing strand. Mismatches may be tolerated to some extent, as long as in the circumstances, the stretch of nucleotides is capable of hybridizing to its complementary part.

The nucleic acid molecule interfering specifically with dynamin 2 expression is preferably an antisense nucleic acid, an RNAi nucleic acid, or a ribozyme interfering specifically with dynamin 2 expression.

The term *"antisense nucleic acid*" or *"antisense oligonucleotides"* (ASO) designates a synthetic single-stranded oligonucleotide of which the sequence is at least partially complementary to a target nucleic acid, such as to a pre-mRNA or a mRNA sequence of a target gene (Lee et al., J Cardiovasc Transl Res. 2013; 6(6):969-80.; DeVos et al., Neurotherapeutics 2013; 10(3):486-972013). An antisense nucleic acid is capable of altering the expression of a specific target gene, either by splicing modification such as by inducing exon-skipping, or by recruiting RNAse H leading to RNA degradation of RNA-DNA duplex, thus blocking the expression of the target gene. An antisense nucleic acid is typically short in length, in general 5 to 50 nucleotides in length, such as 5, 10, 15, 20, 25, 30, 35, 40, 45 or 50 nucleotides in length, more preferably 15, 20, 25, 30, 35 nucleotides in length. It is well within the skill of the person in the art to design antisense nucleic acids specific to a target, based on the knowledge of a target sequence such as introns, exons, 5'CAP or pre-mRNA sequences (DeVos et al., Neurotherapeutics 2013; 10(3):486-972013). Antisense nucleic acids can be prepared by methods well-known in the art, such as by chemical synthesis and enzymatic ligation reactions.

In the context of the present invention, an antisense nucleic acid can be used to down-regulate the expression of dynamin 2. Said antisense nucleic acid can be complementary to all or part of a sense nucleic acid encoding dynamin 2, e.g., complementary to the coding strand of a double-stranded cDNA molecule or complementary to an mRNA sequence, and it is thought to interfere with the translation of the target mRNA.

In a preferred embodiment, the nucleic acid molecule interfering specifically with dynamin 2 expression is an antisense nucleic acid that is complementary to at least a part of the mRNA or pre-mRNA encoding dynamin 2.

For example, the nucleic acid molecule interfering specifically with dynamin 2 expression can be an antisense nucleic acid that is complementary to at least one intron or to the untranslated region (UTR) of the pre-mRNA encoding dynamin 2. The targeted region can be entirely within an intron or UTR of the *DNM2* pre-mRNA, or span an intron/exon junction, or cover at least 50% within an intron of the *DNM2* pre-mRNA.

In a preferred embodiment, the nucleic acid molecule interfering specifically with dynamin 2 expression is an antisense nucleic acid complementary to at least one intron or the 3'UTR of dynamin 2 pre-mRNA, preferably to at least one intron of dynamin 2 pre-mRNA.

In a preferred embodiment the nucleic acid molecule interfering specifically with dynamin 2 expression is an antisense nucleic acid comprising or consisting of at least 8 (preferably at least 8 to 12) contiguous nucleobases of any one of SEQ ID NO: 1 to 3128, even more preferably comprises or consists of any one of SEQ ID NO: 1 to 3128.

In a more preferred embodiment, the nucleic acid molecule interfering specifically with dynamin 2 expression is an antisense nucleic acid complementary to at least intron 12, intron 13, intron 11, intron 1, or intron 14 of dynamin 2 pre-mRNA of dynamin 2 pre-mRNA, more preferably is an antisense nucleic acid comprising or consisting of at least 8 to 16 contiguous nucleobases of any one of the sequences SEQ ID NO: 2873, SEQ ID NO: 3050, SEQ ID NO: 2117, SEQ ID NO: 2183, SEQ ID NO: 2447, SEQ ID NO: 2154 or SEQ ID NO: 2226, even more preferably comprises, or consists, of any one of the sequences SEQ ID NO: 2873, SEQ ID NO: 3050, SEQ ID NO: 2117, SEQ ID NO: 2183, SEQ ID NO: 2447, SEQ ID NO: 2154 or SEQ ID NO: 2226. As reported by WO2020028844 (incorporated by reference in its entirety), the latter antisense nucleic acids exhibit the best properties among SEQ ID NO: 1 to 3128.

Yet, in a more preferred embodiment, the nucleic acid molecule interfering specifically with dynamin 2 expression is an antisense nucleic acid complementary to at least intron 12 of dynamin 2 pre-mRNA, more preferably is an antisense nucleic acid comprising or consisting of at least 8 to 16 contiguous nucleobases of the sequence SEQ ID NO: 2873 or SEQ ID NO: 2117, even more preferably comprises, or consists, of the sequence SEQ ID NO: 2873 or SEQ ID NO: 2117.

Still, in an even more preferred embodiment, the nucleic acid molecule interfering specifically with dynamin 2 expression is an antisense nucleic acid complementary to at least intron 12 of dynamin 2 pre-mRNA, more preferably is an antisense nucleic acid comprising or consisting of at least 8, 9, 10, 11, 12, 13, 14, 15, 16 contiguous nucleobases of the sequence SEQ ID NO: 2873 even more preferably comprises, or consists, of the sequence SEQ ID NO: 2117.

As described above, the antisense nucleic acid may further comprise at least one modified sugar, at least one modified internucleotide (or internucleoside) linkage, and/or at least one modified nucleobase, so as to enhance its properties, such a reduction in sensitivity to nucleases, increase in stability, half-life and/or tissue distribution, and/or enhancement of its affinity towards its target nucleic acid.

In a preferred embodiment, the nucleic acid molecule interfering specifically with dynamin 2 expression is an antisense nucleic acid, as described herein, that comprises at least one modified sugar, in particular in position 2' of the nucleotide (or nucleoside) such as 2'-O-methyl (2'-O-Me), 2'fluoro (2'-F), 2'O-methoxyethyl (2'-MOE) and 2',4'-brigded.

Particularly preferred modified sugars are bicyclic sugars, such as a cEt (constrained ethyl) bicyclic sugar, an LNA bicyclic sugar, or an ENA bicyclic sugar.

*"Bicyclic sugar"* or *"bicyclic sugar moiety"* means a modified sugar comprising two rings, wherein the second ring is formed via a bridge connecting two of the atoms in the first ring thereby forming a bicyclic structure.

A particularly preferred modified sugar according to the invention is cEt bicyclic sugar (constrained ethyl bicyclic sugar). This modified sugar is a bicyclic sugar moiety, wherein the first ring of the bicyclic sugar moiety is a ribosyl sugar moiety, the second ring of the bicyclic sugar is formed via a bridge connecting the 4'-carbon and the 2'-carbon (2',4'-brigded), the bridge has the formula 4'-CH(CH₃)-0-2', and the methyl group of the bridge is in the S configuration. A cEt bicyclic sugar moiety is in the β-D configuration.

In a preferred embodiment, the nucleic acid molecule interfering specifically with dynamin 2 expression is an antisense nucleic acid, as described herein, that comprises at least one modified internucleotide (or internucleoside) linkage, such as a phosphorothioate internucleotide (or internucleoside) linkage.

A *"modified internucleotide linkage"* (or modified internucleoside linkage) is an internucleotide (or internucleoside) linkage in a nucleic acid that is non-naturally occurring, and is thus referred as a non-phosphate linkage. Phosphorothioate linkage is a modified phosphate linkage in which one of the non-bridging oxygen atoms is replaced with a sulfur atom.

In a preferred embodiment, the nucleic acid molecule interfering specifically with dynamin 2 expression is an antisense nucleic acid, as described herein, that comprises at least one modified nucleobase, such as 5-methylcytosine (cytosine is methylated in 5' position).

In a preferred embodiment, the nucleic acid molecule interfering specifically with dynamin 2 expression is an antisense nucleic acid, as described herein, that is a gapmer.

A *"gapmer"* refers to a nucleic acid molecule comprising an internal segment having a plurality of nucleotides (or nucleosides) that support RNase H cleavage positioned between external segments, each having one or more nucleotides (or nucleosides), wherein the nucleotide (or nucleosides) comprising the internal segment are chemically distinct from the immediately adjacent nucleotide(s) (or nucleoside(s)) comprising the external segments. The internal or central segment may be referred to as the *"gap"* or *"gap segment"* and the external segments may be referred to as the *"wings"* or *"wing segments".* Typically, such gapmer can comprise a gap segment of 5 to 15 deoxynucleotides (or deoxynucleosides) flanked by wing segments of 2 to 10 modified nucleotides (or nucleosides).

For example, the nucleic acid molecule interfering specifically with dynamin 2 expression is an antisense nucleic acid, as described herein, that is a gapmer comprising:
a gap segment consisting of linked 2'-deoxynucleotides (or 2'-deoxynucleosides),
a 5' wing segment consisting of linked nucleotides (or nucleosides), and
a 3' wing segment consisting of linked nucleotides (or nucleosides),
wherein the gap segment is positioned between the 5' wing segment and the 3' wing segment and wherein each nucleotide (or nucleoside) of each wing segment comprises a modified sugar, such as a cEt bicyclic sugar.

It should be understood that any of the above modifications that can improve the properties of the antisense nucleic acid can be combined together.

For example, in a preferred embodiment, the nucleic acid molecule interfering specifically with dynamin 2 expression is an antisense nucleic acid that is 16 to 50 nucleobases in length and comprising any one of the sequences SEQ ID NO: 2873, SEQ ID NO: 3050, SEQ ID NO: 2117, SEQ ID NO: 2183, SEQ ID NO: 2447, SEQ ID NO: 2154 or SEQ ID NO: 2226, wherein the antisense further comprises:
a gap segment consisting of 10 linked 2'-deoxynucleotides (or 2'-deoxynucleosides);
a 5' wing segment consisting of 3 linked nucleotides (or nucleosides); and
a 3' wing segment consisting of 3 linked nucleotides (or nucleosides);
wherein the gap segment is positioned between the 5' wing segment and the 3' wing segment, wherein each nucleotide (or nucleoside) of each wing segment comprises a cEt sugar, wherein each internucleotide (or internucleoside) linkage is a phosphorothioate linkage, and wherein each cytosine is a 5-methylcytosine.

In a more preferred embodiment, the nucleic acid molecule interfering specifically with dynamin 2 expression is an antisense nucleic acid comprising or consisting of any one of the following sequences: Gks Tks Tks Tds Ads Tds Tds Ads Tds Ads Gds Gds Gds mCks Tks Tk ( SEQ ID NO: 3129); Gks Gks Aks Tds Tds Tds Tds Ads Gds Gds Ads Gds Gds Tks Gks Ak (SEQ ID NO: 3130); Gks mCks Aks Tds Ads Gds Ads mCds Ads Ads Ads Tds mCds mCks mCks Ak (SEQ ID NO: 3131); Gks mCks Aks Ads Ads Tds Ads Tds Gds Ads Tds Tds mCds Aks Tks mCk (SEQ ID NO: 3132); Gks Gks Tks mCds Ads Tds Tds Ads Ads Ads Gds Ads Tds Tks mCks Tk (SEQ ID NO: 3133); Aks Tks Gks Tds Ads Tds Tds Ads mCds mCds Tds Ads mCds Gks Gks mCk (SEQ ID NO: 3134); or Gks Tks Aks mCds Ads Ads Tds Gds Tds Ads Ads Gds mCds mCks Tks Tk (SEQ ID NO: 3135); wherein A is an adenine, mC is a 5-methylcytosine, G is a guanine, T is a thymine, k is a cEt sugar moiety, d is a 2'-deoxyribosyl sugar moiety, and s is a phosphorothioate internucleotide (or internucleoside) linkage.

In an even more preferred embodiment, the nucleic acid molecule interfering specifically with dynamin 2 expression is an antisense nucleic acid comprising or consisting of any one of the following sequences: Gks Tks Tks Tds Ads Tds Tds Ads Tds Ads Gds Gds Gds mCks Tks Tk (SEQ ID NO: 3129); Gks mCks Aks Tds Ads Gds Ads mCds Ads Ads Ads Tds mCds mCks mCks Ak (SEQ ID NO: 3131); wherein A is an adenine, mC is a 5-methylcytosine, G is a guanine, T is a thymine, k is a cEt sugar moiety, d is a 2'-deoxyribosyl sugar moiety, and s is a phosphorothioate internucleotide (or internucleoside) linkage.

In the most preferred embodiment, the nucleic acid molecule interfering specifically with dynamin 2 expression is an antisense nucleic acid comprising or consisting of the sequence Gks Tks Tks Tds Ads Tds Tds Ads Tds Ads Gds Gds Gds mCks Tks Tk (SEQ ID NO: 3129); wherein A is an adenine, mC is a 5-methylcytosine, G is a guanine, T is a thymine, k is a cEt sugar moiety, d is a 2'-deoxyribosyl sugar moiety, and s is a phosphorothioate internucleotide (or internucleoside) linkage.

Alternatively, an antisense nucleic acid can be designed to block a splice acceptor (SA) site and/or an exon splicing enhancer (ESE) and/or a branch point in a pre-mRNA and/or any sequence which could modulate pre-mRNA splicing, i.e. it can be designed to be complementary to a part of the pre-mRNA comprising an SA, an ESE, a branch point sequence or any sequence which could modulate pre-mRNA splicing.

For example, an antisense nucleic acid can be designed to induce exon-skipping within a pre-mRNA, thereby leading to a frameshift which produces a truncated cDNA containing a premature stop codon in the resulting mRNA. This strategy can be applied herein to the *DNM2* pre-mRNA, so as to reduce the level of DNM2 protein.

Accordingly, in a preferred embodiment, the nucleic acid molecule interfering specifically with dynamin 2 expression is an antisense nucleic acid that induces exon-skipping within a dynamin 2 pre-mRNA, such as exon 2 or exon 8 skipping. Skipping of exon 2 or exon 8 was indeed shown to lead to an absence of the dynamin 2 protein (Cowling et al., J Clin Invest. 2014;124(3): 1350-63; Tinelli et al., Hum Mol Genet. 2013; 22(21):4417-29).

Non-limiting examples of antisense nucleic acids inducing exon-skipping within a dynamin 2 pre-mRNA include, without limitation, those comprising or consisting of the sequence SEQ ID NO: 3136 (which induces DNM2 exon 2 skipping), or SEQ ID NO: 3137 (which induces DNM2 exon 8 skipping). To this end, said sequences may preferably be introduced within a U7 small nuclear RNA (U7 snRNA).

Other examples of antisense nucleic acids interfering specifically with dynamin 2 expression are those described in WO2018/189208, incorporated herein by reference in its entirety.

In another embodiment, RNAi can be used to down-regulate the expression of dynamin 2.

*"RNAi nucleic acid*" refers to a nucleic acid that can inhibit expression of a target gene by RNA interference (RNAi) mechanism. By contrast to antisense nucleic acids, RNAi nucleic acids target mature messenger RNA (mRNA). RNAi nucleic acids are well-known in the art, and include short-hairpin RNA (shRNA), small interfering RNA (siRNA), double-stranded RNA (dsRNA), and single-stranded RNA (ssRNA) (Sohail et al. 2004, Gene Silencing by RNA Interference: Technology and Application 1st Edition, ISBN 9780849321412; WO 99/32619; Wang et al., Pharm Res 2011, 28:2983-2995). RNA interference designates a phenomenon by which dsRNA specifically suppresses expression of a target gene at post-transcriptional level. In normal conditions, RNA interference is initiated by double-stranded RNA molecules (dsRNA) of several thousands of base pair length. *In vivo,* dsRNA - such as shRNA- introduced into a cell is cleaved by Dicer into a mixture of short interfering RNA called siRNA; the latter will bind to another enzyme (RISC) that will catalyze the cleavage of both the siRNA and target mRNA (Bernstein et al. Nature. 2001;409(6818):363-6). In mammalian cells, the siRNAs that are naturally produced by Dicer are typically 21-23 bp in length, with a 19 or 20 nucleotides duplex sequence, two-nucleotide 3' overhangs and 5'-triphosphate extremities (Zamore et al. Cell. 2000,101(1):25-33; Elbashir et al. Genes Dev. 2001, 15(2): 188-200; Elbashir et al. EMBO J. 2001, 20(23):6877-88). siRNA or shRNA are usually designed against a region 19 to 50 nucleotides downstream the translation initiator codon, whereas 5'UTR (untranslated region) and 3'UTR are usually avoided. The selected siRNA or shRNA target sequence should be subjected to a BLAST search against EST database to ensure that the only desired gene is targeted. Various products are commercially available to aid in the preparation and use of synthetic siRNA or shRNA. The RNAi nucleic acid can be of at least about 10 to 40 nucleotides (or nucleosides) in length, preferably about 15 to 30 base nucleotides (or nucleosides) in length. siRNA or shRNA can comprise naturally occurring RNA, synthetic RNA, or recombinantly produced RNA, as well as altered RNA that differs from naturally-occurring RNA by the addition, deletion, substitution and/or alteration of one or more nucleotides (or nucleosides). Such alterations can include addition of non-nucleotide (or non-nucleoside) material, such as to the end of the molecule or to one or more internal nucleotides (or nucleoside) of the siRNA, including modifications that make the siRNA resistant to nuclease digestion, as described above. Particularly preferred modified RNAi nucleic acids are those comprising at least one modified sugar, in particular in position 2' of the nucleotide, such as 2'-O-methyl (2'-O-Me) or 2'fluoro (2'-F), as described in more details below.

Some dynamin 2 RNAi nucleic acids are commercially available. One can cite, for example, those commercialized by Abnova-Novus Biologicals (dynamin 2 RNAi No. H00001785-R05-H00001785-R08), or by Santa Cruz Biotechnology (dynamin II siRNA (h) No. sc-35236; dynamin II (h)-PR No. sc-35236-PR; dynamin II shRNA plasmid (h) No. sc-35236-SH; dynamin II shRNA (h) Lentiviral Particles No. sc-35236-V).

Thus, in a preferred embodiment, the nucleic acid molecule interfering specifically with dynamin 2 expression is an RNAi nucleic acid that is complementary to at least one part of DNM2 mRNA, in particular to at least one exon. The RNAi nucleic acid can be a siRNA or shRNA of at least about 10 to 40 nucleotides (or nucleosides) in length, preferably of about 15 to 30 nucleotides (or nucleosides) in length.

More preferably, the nucleic acid molecule interfering specifically with dynamin 2 expression is an RNAi nucleic acid that is complementary to at least exon 1, 4, 5, 12b, 13, 15, 17 and/or 21 of dynamin2 mRNA.

Even more preferably, the nucleic acid molecule interfering specifically with dynamin 2 expression is an RNAi nucleic acid comprising or consisting of a sequence selected from any one of SEQ ID NO: 3138 to SEQ ID NO: 3151.

Other examples of RNAi nucleic acids interfering specifically with Dynamin 2 expression are allele-specific siRNA described in WO2018/100010, incorporated herein by reference in its entirety.

In another embodiment, the nucleic acid interfering specifically with dynamin 2 expression is a ribozyme.

*"Ribozymes"* are catalytic RNA molecules with ribonuclease activity which are capable of cleaving a single-stranded nucleic acid, such as an mRNA, to which they have a complementary region. Thus, ribozymes can be used to catalytically cleave mRNA transcripts, thereby inhibiting translation of the protein encoded by the mRNA. Ribozyme molecules specific for a target (herein dynamin 2) can be designed, produced, and administered by methods commonly known to the art (see e.g., Fanning and Symonds (2006) RNA Towards Medicine (Handbook of Experimental Pharmacology), ed. Springer p. 289-303).

Yet, in another embodiment, genome editing can be used to inhibit dynamin 2. More specifically, one can use a genome editing system comprising a nuclease engineered to target the *Dnm2* gene.

*"Genome editing"* is a type of genetic engineering in which DNA is inserted, replaced, or removed from a genome using artificially engineered nucleases, also called molecular scissors. Nucleases create specific double-stranded break (DSBs) at desired locations in the genome, and harness the cell's endogenous mechanisms to repair the induced break by natural processes of homologous recombination (HR) or non-homologous end-joining (NHEJ). There are currently four known families of nucleases suitable for genome editing: zinc finger nucleases (ZFNs), transcription activator-like effector nucleases (TALENs), the CRISPR/Cas system in particular the Cas9 system (Mali et al, Nature Methods, 2013;10(10):957-63), or engineered meganucleases re-engineered homing endonucleases. Said nucleases can be delivered to the cells either as DNAs or mRNAs, such DNAs or mRNAs and can be engineered to target the *Dnm2* gene.

A particularly preferred genome editing system according to the invention is the CRISPR/Cas system in particular the Cas9 system.

In another embodiment, the dynamin 2 inhibitor used in the present invention is an antibody directed against dynamin 2.

An *"antibody"* is a polypeptide capable of specifically recognizing an antigen (herein, dynamin 2). To do so, the antibody's paratope interacts with the antigen's epitope. An antibody typically consists of four polypeptides - two full-length light chains and two full-length heavy chains - which are joined to one another with disulfide bonds to form a Y-shaped protein. Herein, the term antibody encompasses as well antibody fragments (Fab, Fv, ScFv, HCAb, sdAb, etc.) and antibody mimetics (ABDs, adhirons, affibodies, affimers, armadillo repeat proteins, DARPins, pronectins, transbodies, trimers X, etc.). This term also encompasses humanized and chimeric antibodies. Methods for preparing, using, and characterizing antibodies as defined herein are well known in the art (Immunobiology by Janeway et al., 5th edition, Garland publishing, 2001; Therapeutic Monoclonal Antibodies: From Bench to Clinic by An et al., Wiley editions, 2009; Skrlec et al. Trends in Biotechnology; 2015, 33(7): 408-41.; Harlow, E. and Lane, D. (1988) Antibodies: A Laboratory Manual, ed., Cold Spring Harbor Laboratory).

Examples of antibodies directed against dynamin 2 that are available, include, without limitation, antibodies sold or made by Novus Biologicals (catalogue No. NB300-617, NBP2-16244, (6C9) H00001785-M01), by Santa Cruz Biotechnology (catalogue No. sc-81150, sc-6400, sc-166525, sc-166669, sc-166526,) by BD-Biosciences (anti-DNM2 mouse ab, 610264), or by IGBMC-Illkirch (R2679, R2680, R2865, R2866, R2640, or R2641).

In another embodiment, the dynamin 2 inhibitor used in the present invention is a small molecule inhibiting the dynamin 2 enzymatic activity (i.e., inhibition of the GTPase activity), expression (such as by inhibiting promoter, splicing or translation) or function (such as inhibition of oligomerisation, activation, lipid binding, or partner binding).

As used throughout the specification, a *"small molecule"* refers to a low molecular weight compound, typically less than 1000 Daltons, which can be organic or inorganic. A small molecule can be derived from any known organism (including, but not limited to, animals, plants, bacteria, fungi and viruses) or from a library of synthetic molecules.

Small molecules inhibiting dynamin 2 activity, expression or function can be identified with the method described herein. Small molecules that act as dynamin inhibitors are described in Harper et al. (Trends Cell Biol. 2013 Feb;23(2):90-101).

Examples of small molecules inhibiting dynamin 2 activity, expression or function that can be used according to the invention, include, without limitation, Dynasore (a noncompetitive, cell-permeable semicarbazone compound inhibitor of dynamin 1 and dynamin 2; CAS number 304448-55-3; chemical name: 3-hydroxynaphthalene-2-carboxylic acid (3,4-dihydroxybenzylidene)hydrazide); hydroxy-Dynasore (a highly potent inhibitor of dynamin 2 having a IC50 = 2.6 µM, which is a cell-permeable hydroxylated analog of Dynasore; CAS number 1256493-34-1; chemical name: 3-hydroxy-N'-[(2,4,5-trihydroxyphenyl) methylidene]naphthalene-2-carbohydrazide); tetradecyltrimethylammonium bromide (CAS number 1119-97-7; sold under the name MiTMAB^{™} (ab 120466) by Abcam; it is a cell permeable dynamin 1 and dynamin 2 inhibitor with an IC50 = 8.4 µM re. dynamin 2; it targets the pleckstrin homology domain and inhibits receptor-mediated and synaptic vesicle endocytosis); phthaladyn-23 (a cell-permeable phthalimide compound that inhibits dynamin 2 GTPase activity with an IC50 = 63 µM ; chemical name: 4-chloro-2-((2-(3-nitrophenyl)-1,3-dioxo-2,3-dihydro-1H-isoindole-5-carbonyl)-amino)-benzoic acid); Dynole 34-2 (a dynamin inhibitor V that acts on GTPase activity, noncompetitive for GTP; chemical name: 2-cyano-N-octyl-3-[1-(3-dimethylaminopropyl)- 1 H-indol-3 -yl] acrylamide), m-divi 1 (a mitochondrial division inhibitor with IC50 = 10µM ; chemical name: 3-(2,4-Dichloro-5-methoxyphenyl)-2-sulfanylquinazolin-4(3H)-one); iminodyn-22 (IC50 = 390nM; acts on a GTPase allosteric site and displays uncompetitive antagonism with respect to GTP; chemical name: N,N'-(Propane-1,3-diyl)bis(7,8-dihydroxy-2-imino-2H-chromene-3-carboxamide); iminodyn 17 (the chemical name: N,N'-(Ethane-1,2-diyl)bis(7,8-dihydroxy-2-imino-2H-chromene-3 -carboxamide); OcTMAB (it targets the PH domain of dynamin 2; chemical name: octadecylTriMethylAmmonium bromide); the dynamin inhibitory peptide with amino acid sequence QVPSRPNRAP (Tocris Biosciences 1774); Dyngo-4a (IC50 -2.5µM ; it acts on a GTPase allosteric site; chemical name :3-Hydroxy-N'-[(2,4,5-trihydroxyphenyl) methylidene]naphthalene-2-carbohydrazide); RTIL-13 (IC50 -2.3µM ; it is a norcantharidin scaffold targeting the PH domain of dynamin 2; chemical name: 4-(N,N-Dimethyl-N-octadecyl-N-ethyl)-4-aza-10-oxatricyclo-[5.2.1]decane-3,5-dione bromide).

The therapy for use according to the invention combines (i) any one of the dynamin 2 inhibitors as described above with (ii) any therapy providing or restoring a functional dystrophin in a subject.

The term *"dystrophin"* refers a rod-shaped protein which connects the extracellular matrix and the cytoskeleton, thereby supporting muscle fiber strength. As explained above, dystrophin is naturally encoded in humans by the *DMD* gene. Full-length human dystrophin has a molecular weight of about 427 kb. Three full-length isoforms of dystrophin, having the same number of exons, can actually be expressed in either human brain, muscle and Purkinje cerebellar neurons via three independent promoters. In the context of the present invention, it should be understood that the dystrophin of interest is one that can be expressed in muscle, more particularly in skeletal muscles and cardiomyocytes. The native sequence of full-length human dystrophin that is mainly expressed in muscles is well-known in the art (NCBI database amino acid sequence: NP_003997.2; coded by mRNA sequence: NM_004006.3). Numerous mutations can affect the *DMD* gene, such as exonic frameshift, deletions, substitutions, nonsense mutations, and duplicative mutations, which can diminish the expression of dystrophin, thereby leading to dystrophinopathies. It is therefore desired to compensate or treat these mutations via a therapeutic product that can provide the affected subject with a functional dystrophin. It is within the skill of the person in the art to identify the *DMD* mutation in the subject to be treated, using techniques well-known in the art such as multiple PCR, MLPA (multiplex ligation-dependent probe amplification), and sequencing (Sun et al., Genes (Basel). 2020; 11(8): 837). For guidance, databases reporting all mutations identified in patients are also available, such as the Leiden DMD mutation database (Aartsma-Rus et al., Muscle Nerve 2006; 34(2):135-44).

The term *"functional dystrophin"* encompasses herein the full-length wildtype dystrophin as described above as well as shortened forms, fragments, or variants thereof that retain all or part of the dystrophin function, such as at least all or part of the capacity to bind to actin (typically provided by the N-terminal domain) and/or to the dystrophin associated glycoprotein complex (DGC). Binding of dystrophin to actin and to the DGC complex may be visualized by either co-immunoprecipitation using total protein extracts or immunofluorescence analysis of cross-sections, from a biopsy of a muscle suspected to be dystrophic, as known to the skilled person. To do so, one can for example rely on animal models of dystrophin-related myopathy such as the dystrophin-deficient mouse (*mdx*) (Ahmad et al., Hum Mol Genet 2000, 9: 2507-2515; Allamand et al. Hum Mol Genet 2000, 9: 2459-2467; Mann et al. Proc Natl Acad Sci U S A 2001, 98: 42-47; Wu et al., Am J Pathol 2012, 181: 392-400), the dystrophin/utrophin double knock-out mouse (*DKO*) (Rafael et al., Hum Mol Genet 2000, 9: 1357-1367), the dystrophic dog (*grmd*) (Sharp et al. Genomics 1992, 13: 115-121; Howell et al., Neuromuscul Disord 1997, 7: 325-328), the transgenic pig (Klymiuk al. Hum Mol Genet 2013, 22(21): 4368-82), or the transgenic humanized *hDMD* mouse (Bremmer-Bout al. Mol Ther 2004, 10: 232-240).

In the context of the present invention, *"a therapy providing a functional dystrophin"* typically refers to a therapy wherein an exogenous dystrophin that is functional is provided to the subject while *"a therapy restoring a functional dystrophin"* generally relates to a therapy that restores the expression of the endogenous dystrophin of the subject so that said dystrophin is functional.

The scientific and patent literature have reported a wide range of means to provide or restore a functional dystrophin in a subject (Sun et al., Genes (Basel). 2020, 11(8): 837; WO2009/054725; all incorporated herein by reference in their entirety). Below is provided a non-exhaustive list of such means which can be used in the present invention.

In a preferred embodiment, the therapy providing or restoring a functional dystrophin in a subject is selected from the group consisting of: dystrophin gene therapy, exon-skipping therapy, codon-read-through therapy, genome editing therapy, myogenic cell therapy, and any combinations thereof.

In a preferred embodiment, one may wish to provide a functional dystrophin by gene replacement therapy, such as by vector-mediated gene therapy.

This approach is suited to treat any anomaly of the *DMD* gene, i.e. all dystrophin-related myopathies. Although this approach seemed initially challenging because of the huge size of the dystrophin gene and the widespread distribution of muscles, it is now fully possible to provide a truncated dystrophin gene that remains functional, or even the full-length dystrophin gene (i.e. functional), typically via vectors such as adeno-associated viruses (AAV) or human artificial chromosomes (HAC). Micro-dystrophin genes are generally about 4 kb in length, without the C-terminal domain; while mini-dystrophin genes are generally about 6 kb in length and include all functional domains. Micro-dystrophin and mini-dystrophin genes are *de facto* functional. The full-length dystrophin gene may preferably be provided via multiple independent vectors, such as via a transplicing vector system.

Accordingly, in a preferred embodiment, the gene therapy provides a functional dystrophin selected from a mini-dystrophin, a micro-dystrophin, or a full-length dystrophin.

To do so, one can use a vector comprising a nucleic acid sequence encoding said functional dystrophin.

Such functional dystrophins have been widely described in the scientific and patent literatures, and are thus well-known in the art (Harper et al. Nat Med. 2002, 8(3):253-61; Kornegay et al. Mol. Ther. J. Am. Soc. Gene Ther. 2010, 18:1501-1508; Kazuki et al. Gene Ther. 2011, 18: 384-393; Bowles et al. Mol. Ther. 2012, 20: 443-455; Le Guiner et al. Nat. Commun. 2017, 8: 16105; Duan et al., Mol. Ther. 2018, 26: 2337-2356; Sinenko et al., Exp. Cell Res. 2020, 389: 111882; Sun et al., Genes 2020, 11:837; the contents of which are incorporated herein by reference in their entireties).

Examples of micro-dystrophins that are suitable according to the invention, include, without limitation, the micro-dystrophins ΔDysM3 (Yuasa et al. Nihon Rinsho. 1997; 55:3148-3153); ΔDysH1, ΔDysH4, ΔDysHAH3, ΔDysAX2, ΔDysAX11 (Yuasa et al., FEBSLett. 1998; 425:329-336); Δ3447, Δ3510, Δ3531, Δ3849, Δ3990, Δ4173 (Wang et al., Proc. Natl. Acad. Sci. USA. 2000, 97:13714-13719; WO01/83695); ΔR1-R24, ΔR4-23/ΔCT (also named ΔCS1, MD1, or H2µDys), ΔR4-R23 (also named CS1), ΔR2-R21,ΔR2+R21+H3 (Harper et al. Nat. Med. 2002; 8:253-261; Sakamoto et al. Biochem. Biophys. Res. Commun. 2002; 293:1265-1272; Yoshimura et al. Mol. Ther. 2004; 10:821-828.); Δ3788 (also named ΔAB/ΔR3-18/ΔCT; Fabb et al. Hum. Mol. Genet. 2002; 11:733-741); ABS1µDys, ABS1.2µDys (Banks et al. Hum. Mol. Genet. 2007; 16:2105-2113); ΔR2-15/ΔR18-23/ΔC (also named R16-17/ΔC or YL90), ΔR3-15/ΔR18-23/ΔC (also named YL93), ΔR3-15/ΔR17-23/ΔC (also named YL113) (Lai et al., J. Clin. Invest. 2009; 119:624-635); M1, M2, M3, M4 (Jorgensen et al. Hum. Gene Ther. 2009; 20:641-650); ΔH2-R24/ΔCT, ΔH2-R23+H3/ΔCT (also named H3µDys), ΔH2-R23+R18-H3/ΔCT, ΔR4-R23/ΔCT/ΔpolypP (Banks et al. PLoS Genet. 2010; 6:e1000958); MD1, MD2 (Koo et al. Hum. Gene Ther. 2011; 22:1379-1388); ΔCS2 (Shin et al. Gene Ther. 2011; 18:910-919); R6-17/H3/ΔC (also named ΔR2-15/ΔR18-19/ΔR20-23/ΔC, or YL196; Shin et al. Hum. Gene Ther. 2012; 23:202-209); mDys5R (also named ΔR2-15/ΔR18-19/ΔR20-22/ΔC, or XP42 or µDys5R; Hakim et al. Mol. Ther. Methods Clin. Dev. 2017; 6:216-230; WO2008/088895 and WO2016115543) (the contents of these publications and patent applications being incorporated herein by reference in their entireties).

Particularly preferred micro-dystrophins to be used according to the invention are micro-dystrophins Δ3990, ΔR4-23/ΔCT, ΔR4-R23/ΔCT/ΔpolypP (ΔR4-23/ΔCT in which the polyproline has been deleted in hinge 2), R16-17/ΔC and µDys5R, more preferably the micro-dystrophins Δ3990, ΔR4-23/ΔCT, and µDys5R. Common features of micro-dystrophins Δ3990, ΔR4-23/ΔCT, ΔR4-R23/ΔCT/ΔpolypP and µDys5R are the presence of the N-terminal domain, the cysteine-rich domain, spectrin-like repeats 1 and 24, hinges 1 and 4, as well as the absence of the C-terminal domain. The differences between micro-dystrophins Δ3990, ΔR4-23/ΔCT, ΔR4-R23/ΔCT/ΔpolypP and µDys5R are in the central hinges and R16-17 nNOS-binding domain. Δ3990 micro-dystrophin contains hinge 3, while ΔR4-23/ΔCT and ΔR4-R23/ΔCT/ΔpolypP micro-dystrophins contains hinge 2, and µDys5R micro-dystrophin has no central hinge. Only µDys5R and R16-17/ΔC carry the R16-17 nNOS micro-dystrophin domain.

Alternatively, one may use a mini-dystrophin such as the Δ17-48 mini-dystrophin, though the packaging of its corresponding nucleic sequence in a vector, in particular in an AAV vector, might be more challenging than for mini-dystrophins due to its larger size (6.2 kb) (England et al. Nature. 1990; 343:180-182; Chamberlain et al. Hum. Mol. Genet. 2002; 11:2355-236).

Another possibility is to provide the full-length dystrophin gene via e.g. multiple independent vectors, such as via transplicing vector system. In a transplicing system, such as an AAV transplicing vector system, the vectors typically carry sequential parts of the *DMD* gene that can be co-joined to generate the full-length protein through trans-splicing events (Koo et al., Hum. Gene Ther. 2014, 25: 98-108).

In order to increase the expression level of the functional dystrophin provided by gene therapy, one may proceed to codon optimization of the nucleic acid sequence encoding said dystrophin according to methods conventional in the art (Foster et al. Mol Ther. 2008, 16(11): 1825-32; Athanasopoulos et al. Methods Mol Biol. 2011, 709:21-37). An example of an optimized micro-dystrophin gene is opti-DysΔ3978, also referred as Δ3978 micro-dystrophin gene or DysΔ3978: it encodes the same protein as the Δ3990 micro-dystrophin gene but with optimized DNA sequences for better RNA processing and protein translation (Kornegay et al., Mol Ther. 2010, 18(8): 1501-1508). See also WO2017221145, incorporated herein by reference in its entirety.

The skilled person in the art may also wish to select particular promoters to drive the expression of the transgene, either ubiquitously or in specific cell types (herein, muscle, such as skeletal and/or cardiac muscle). Ubiquitous promoters or promoters having a low tissues specificity include, without limitation, the phosphoglycerate kinase 1 (PGK) promoter, EF1 promoter, b-actin promoter, or CMV promoter. Promoters that are muscle-specific include, without limitation, the desmin promoter, muscle creatine kinase (MCK) promoter, MHCK7 promoter, CK6 promoter, CK8 promoter, Syn promoter, or C5-12 (spC5-12, or Sp5.12) promoter. The latter allows a robust expression in skeletal and cardiac muscles. Muscle-specific promoters are herein particularly preferred.

As explained above, vectors are particularly suitable to deliver a functional dystrophin by gene therapy.

In its broadest sense, *"vector"* means any vehicle capable of facilitating the transfer of a molecule in a cell, tissue or host, and allow or promote its expression, activity or function within the cell, tissue or host. Such vectors include, but are not limited to, plasmids, phagemids, viruses, and other vehicles derived from viral or bacterial sources, or even human sources such as chromosomes, as these can be genetically modified. The term vector also encompasses hydrogels, cyclodextrins, nanoparticles, microspheres, and any vehicles allowing to target a cell, tissue or host such as a cell-penetrating peptide or an antibody (such as one targeting the transferrin receptor).

Viral vectors are particularly preferred vectors as many of them have been validated for clinical applications, notably for gene therapy; these include, without limitation, adeno-associated vectors (AAV), adenoviral vectors, baculoviral vectors, herpes viral vectors, retroviral vectors such as lentiviral vectors.

Preferred vectors suitable for providing a functional dystrophin by gene therapy according to the invention are viral vectors and artificial chromosomes.

Among viral vectors, AAV vectors are particularly suited for gene therapy, as they can accommodate about 5 kb, are moderately immunogenic and noncytopathic, and can be specifically targeted to one or more types of cells by choosing the appropriate AAV serotype or combination of serotypes. Because of their limited packaging capacity, AAV vectors are more suited for micro-dystrophin genes. It is nevertheless possible today, as explained above, to use an AAV transplicing vector system so as to provide the full-length dystrophin, or a mini-dystrophin.

Particularly preferred AAV vectors to be used according to the invention are those capable of transducing muscle cells. These include, without limitation, the AAV recombinant vectors of serotype 1, 2, 5, 8 or 9, or variants (hybrids) thereof such as the AAV2.5 (Bowles et al. Mol. Therapy 2012, 20(2): 443-455), AAV2.6 (US 6,156,303), AAV2.8 (US 7,282,199), AAVrh8 (WO2003/042397), AAV2.9 (WO2005/033321), eAAV9 (Jackson et al., Mol Ther Methods Clin Dev. 2020; 19: 496-506), or AAVrh74 (an AAV8 variant) vectors, to name a few (See also Wang et al., Expert Opin Drug Deliv., 2014;11(3):345-364; Muraine et al., Hum Gene Ther., 2020;31(3-4):233-240; Gernoux et al., Mol Ther., 2020;28(3):747-757).

On the other hand, an artificial chromosome such as a human artificial chromosome (HAC) is more suited to deliver a full-length *DMD* gene (Hoshiya et al., Mol Ther. 2009,17(2):309-17; Kazuki et al., Mol Ther. 2010,18(2):386-93). This type of chromosome is generally derived from a native chromosome by top-down engineering, or by bottom up *de novo* synthesis, and has the capacity replicate during mitosis as an extra genomic copy.

In a more preferred embodiment, the vector providing a functional dystrophin by gene therapy according to the invention is an AAV vector comprising a nucleic acid sequence encoding a mini- or micro-dystrophin, that is preferably selected from the group consisting of the recombinant AAV vectors rAAV2.5-CMV-Δ3990 micro-dystrophin (such as from Nationwide Children's Hospital); rAAV9-CK8-micro-dystrophin (such as the one referred as SGT-001 from Solid Biosciences; the micro-dystrophin contains the nNOS domain and is thus most likely the µDys5 micro-dystrophin); rAAVrh74-MHCK7-micro-dystrophin (such as the one referred as SRP-9001 from Nationwide Children's Hospital/Sarepta Therapeutics, Inc; this micro-dystrophin comprises the N-terminus for binding to f-actin, spectrin repeats 1 to 3 and 24, hinges 1, 2, and 4, and the cysteine-rich domain and thus likely corresponds to the ΔR4-R23/ΔCT micro-dystrophin; see also WO2017181015, WO2018170408, and Mendell et al. JAMA Neurol. 2020 77(9):1122-1131, all incorporated herein by reference in their entireties); rAAV9-muscle specific promoter-Δ3978 micro-dystrophin (such as the one referred as PF-06939926 from Pfizer); rAAV9-Sp5.12-micro-dystrophin (such as the one referred as GNT0004 from Genethon and Sarepta Therapeutics; this micro-dystrophin is likely the ΔR4-23/ΔCT micro-dystrophin; see also WO 2015/197869 incorporated herein by reference in its entirety); and rAAVrh74-MCK-GALGT2 (such as the one from Nationwide Children's Hospital).

In another embodiment, one may wish to restore the expression of endogenous dystrophin by exon-skipping.

Exon-skipping of mutated exons can indeed be particularly useful so as to restore the reading frame of the dystrophin pre-mRNA in patients in need thereof. For instance, by the targeted skipping of a specific exon, a DMD phenotype (lacking dystrophin) can be converted into a milder BMD phenotype (in which the dystrophin is partly to largely functional). As explained above, exon-skipping is preferably induced by nucleic acid molecules in particular by antisense nucleic acids, that can target for example either one or both of the splice sites, or exon-internal sequences. Applied to the dystrophin gene, exon-skipping will result in the expression of a shorter dystrophin protein, of which the function should at least alleviate the symptoms. Such therapeutic strategy can treat about 55% of DMD patients. It is within the skill of the person in the art to generate an exon-skipping product capable of providing a subject with a functional dystrophin, based on the *DMD* mutation identified in the subject to be treated.

Accordingly, in a preferred embodiment, the exon-skipping therapy restoring a functional dystrophin induces exon-skipping of at least one mutated exon of a dystrophin pre-mRNA, such as at least one of mutated exons 1 to 79 of a dystrophin pre-mRNA.

Most common mutated exons identified in DMD patients are found in exons 51, 53, 45, 44, 52, 43, 23, 35, 50, 7, 8, 17 and/or 55 of the dystrophin pre-mRNA, more particularly in exons 51, 53, 45, 44 and/or 43 of the dystrophin pre-mRNA.

Thus, in a preferred embodiment, the exon-skipping therapy restoring a functional dystrophin induces exon-skipping of at least one of exons 51, 53, 45, 44, 52, 43, 23, 35, 50, 52, 7, 8, 17 and/or 55 of a dystrophin pre-mRNA, more preferably of at least one of exons 51, 53, 45, 44 and 43 of a dystrophin pre-mRNA.

To do so, one can use exon-skipping nucleic acid molecules, more particularly antisense nucleic acids.

Antisense nucleic acids inducing exon-skipping of at least one of mutated exons of a dystrophin pre-mRNA are indeed well-known in the art (WO2001083740; WO2002024906; WO2004083446; WO2004048570; WO2006000057; US8232384B2; WO2006021724; WO2007135105; US8361979B2; WO2009054725; US9926557B2; WO2009139630; US9139828B2; WO2010048586; US20100130591; WO2011057350; US8324371B2; WO2012029986; US9079934B2; WO2012109296; US9078911B2; WO2014144978; WO2014153220; US9217148B2; WO2015194520; WO2017062862; US9079934B2; US9034838B2; WO2019060775; WO2020029931 such as the antisense nucleic acids in Table 2; Echigoya et al., Mol Ther. 2017, 25(11):2561-2572 such as the antisense nucleic acids in Table 1; Echigoya et al., Mol Ther. 2019, 27(11): 2005-2017 such as the antisense nucleic acids in Table 2; the contents of which are incorporated herein by reference in their entireties).

Exon-skipping antisense nucleic acids that are suitable for restoring a functional dystrophin according to the invention include, without limitation, eteplirsen (a morpholino phosphorodiamidate antisense nucleic acid comprising the sequence SEQ ID NO: 3152 and inducing exon 51-skipping from *DMD* pre-mRNA; also known as Exondys51 from Sarepta Therapeutics Inc.; full chemical name: RNA, [P-deoxy-P-(dimethylamino)](2',3'-dideoxy-2',3'-imino-2',3'-seco)(2'a→5')(C-m5U-C-C-A-A-C-A-m5U-C-A-A-G-G-A-A-G-A-m5U-G-G-C-A-m5U-m5U-m5U-Cm5U-A-G), 5'-[P-[4-[[2-[2-(2-hydroxyethoxy)ethoxy]ethoxy]carbonyl]-1-piperazinyl]-N,Ndimethylphosphonamidate]); drisapersen (a 2'-O-methyl phosphorothioate antisense nucleic acid comprising the sequence SEQ ID NO: 3153 and targeting the splicing of exon 51 in the *DMD* pre-mRNA, also known as Kyndrisa from BioMarin); SRP50-51 (a morpholino phosphorodiamidate antisense nucleic acid inducing exon 51-skipping from the *DMD* pre-mRNA, which comprises the sequence SEQ ID NO: 3152 and is combined to a cell-penetrating peptide); suvodirsen (a stereopure antisense nucleic acid and inducing exon 51-skipping from *DMD* pre-mRNA; also known as WVE-210201 from Wave Life Sciences); golodirsen (a morpholino phosphorodiamidate antisense nucleic acid comprising the sequence SEQ ID NO: 3154 and inducing exon-skipping of exon 53 *from DMD* pre-mRNA; also known as Vyondys53 from Sarepta Therapeutics Inc.); viltolarsen (morpholino antisense nucleic acid comprising the sequence SEQ ID NO: 3155 and targeting the splicing of exon 53 in the *DMD* pre-mRNA; also known as NS-065/NCNP-01 or Viltepso from Nippon Shinyaku); WVE-5N31 (antisense nucleic acid targeting the splicing of exon 53 in the *DMD* pre-mRNA; Wave Life Sciences); casimersen (a morpholino phosphorodiamidate antisense nucleic acid comprising the sequence SEQ ID NO: 3156 and inducing exon-skipping of exon 45 from DMD pre-mRNA; also known as Amondys45 from Sarepta Therapeutics Inc.); DS-5141b (antisense nucleic acid consisting of 2'-O,4'-C-ethylene-bridged nucleic acid (ENA) and 2'-O-methyl RNA, and targeting the splicing of exon 45 in the *DMD* pre-mRNA; Daiichi Sankyo); NS-089/NCNP-02 (antisense nucleic acid targeting the splicing of exon 44 in the *DMD* pre-mRNA; NS Pharma/Nippon Shinyaku); and any combinations thereof.

In another embodiment, it is possible to restore a functional dystrophin in a subject by selectively inducing ribosomal read-through of premature stop codons that may be present in the dystrophin mRNA of patients. To do so, one may rely on a codon-read-through therapy. By contrast to exon-skipping therapy, codon-read-through therapy does not be to be tailored to each patient. However, such therapy will only correct non-sense mutations.

*"A codon-read-through therapy", "read-through therapy",* or "*translational read-through-inducing therapy"* relies on interaction with the ribosome, which leads to insertion of an alternative amino acid at the point of premature termination codon to allow translational read-through, thereby restoring the synthesis of a full-length protein. In other words, a codon-read-through therapy leads to stop codon suppression. Without being bound by theory, codon-read-through therapy is applicable to all-nonsense mutations (also called PTC for premature termination codon), which represent, in the context of the present invention, about up to 10-15% of DMD patients. Nonsense mutations (PTCs) are generally associated with a dramatic reduction in gene expression. This is because mRNAs containing in-frame PTCs (PTC-mRNAs) are substrates of the nonsense mediated decay pathway (NMD), a mRNA surveillance process that recognizes and rapidly degrades them. Because the expression of PTC genes is compromised by a premature arrest of translation and low level of PTC mRNAs, PTCs preclude the synthesis of a functional full protein, and thus lead to severe inherited diseases.

Small molecules have been identified and also developed for their capacity to restore unfunctional or aberrant PTC containing genes, such as the *DMD* gene, and are thus well-known in the art (Du et al. J. Exp. Med. 2009; 206:2285-2297; Keeling et al. Crit. Rev. Biochem. Mol. Biol. 2012, 47:444-463; Du et al. Mol Ther 2013, 21(9):1653-60; Keeling et al. Annu Rev Genomics Hum Genet 2014, 15: 371-394; Baradaran-Heravi et al. Nucleic Acids Res. 2016, 44(14): 6583-6598; Nagel-Wolfrum et al., BioDrugs. 2016, 30(2):49-74; Namgoong et al. Degener Neurol Neuromuscul Dis 2016, 6:37-48; Dabrowski et al. Mol Med. 2018, 24(1):25; Hamada et al. ACS Med. Chem. Lett. 2019, (10): 1450-1456; Borgatti et al., J Clin Med. 2020, 9(2): 289; Sun et al., Genes 2020, 11:837; the contents of which are incorporated herein by reference in their entireties).

Accordingly, in a preferred embodiment, a codon-read-through therapy restoring a functional dystrophin in the subject comprises a small molecule suppressing premature termination codons (PTC) in a dystrophin mRNA.

Small molecules suppressing premature termination codons that are suitable according to the invention include, without limitation, aminoglycoside compounds such as geneticin (G418), gentamycin, gentamycin B1, paromomycin, amikacin, NB30, NB54, NB74, NB84, NB124, TC007 (a pyramicin); and non-aminoglycoside compounds such as ataluren (3-[5-(2-fluorophenyl)-1,2,4-oxadiazol-3-yl]benzoic acid, also known as PCT124 or Translarna), PCT-414, RTC13 (2-imino-5-{[5-(2-nitrophenyl)-2-furyl]methylene}- 1,3-thiazolidin-4-one), RTC14 (4-tert-butyl-2-[(3-nitrobenzylidene)amino]pheno), GJ071, GJ072, RTC204, RTC219, BZ6, BZ16, negamycin, 3-epideoxynegamycin, leucyl-3-epi-deoxynegamycin, TCP-112, TCP-182, TCP-1109, amlexanox (2-amino-7-isopropyl-5-oxo-5H-chromeno[2,3-b]pyridine-3-carboxylic acid), clitocine (6-amino-5-nitro-4-(ribofuranosylamino)pyrimidine), 2,6-diaminopurine, tylosin, j osamycin, spiramycin, erythromycin, azithromycin; functional analogs thereof; and any combinations thereof; more preferably are gentamycin, paromomycin, ataluren, PCT-414, RTC13, RTC14, and negamycin; even more preferably is ataluren.

In another embodiment, a genome editing therapy can be used to modify an endogenous mutated dystrophin gene so as to restore dystrophin expression in mutated cells of a patient in need thereof. By this approach, one can correct the reading frame of a mutated dystrophin gene.

Thus, in a preferred embodiment, the genome editing therapy restoring a functional dystrophin in the subject comprises a nuclease engineered to target the *Dmd* gene. Genome editing systems comprising a nuclease are as described above, among which the CRISPR/Cas9 system is particularly preferred.

Genome editing therapies for restoring a functional dystrophin are well-known in the art, and are particularly advantageous in that they allow to permanently delete, insert, or replace of one or more exons or aberrant intronic splice acceptor or donor sites within or near the dystrophin gene to be corrected, and thus can durably restore the dystrophin protein function (see for example, to name a few, WO2011141820; WO2014197748; US2016/0201089; WO2016025469; WO2016089866; WO2016161380; WO2017049407; WO2017072590; WO2017095967; WO2018053632; WO2018098480; Young et al. Cell Stem Cell. 2016; 18(4):533-40; Min et al. EN Sci Adv. 2019, 5(3):eaav4324; Zhu et al. Mol Ther Nucleic Acids. 2017, 7:31-41; Babaĉić et al. PLoS One. 2019; 14(2):e0212198; Xu et al. Mol Ther. 2016, 24(3):564-9; Tabebordbar et al., Science. 2016, 351(6271):407-411; the contents of which are incorporated herein in their entireties).

Most genome editing strategies developed to this day to restore dystrophin expression rely on the non-homologous end-joining (NHEJ) mechanism so as to induce exon-skipping.

In another embodiment, one may rely on cellular therapy, wherein the cell expresses a functional dystrophin. Such approach is suited to the treatment of any dystrophinopathy as it stimulates the regeneration of muscles. The cells provided may be from the patient to be treated (autograft), or from a healthy donor (allograft), as long as the cells express or are capable of expressing a functional dystrophin. It is within the skill of the person in the art to isolate, purify, optionally grow and/or differentiate and eventually genetically modify (in the case of an autograft) said cells prior to their administration to the subject to be treated.

In a preferred embodiment, the cell therapy providing the subject with a functional dystrophin is a myogenic cell therapy, comprising for example precursor cells, progenitor cells, or stem cells that are capable of displaying a myogenic phenotype (Biressi et al., J Clin Invest 2020; 130(11):5652-5664).

Precursor cells are unipotent (they can only differentiate into a particular type of cells), whereas progenitor cells and stem cells are multipotent cells (they can differentiate into many cell types). Stem cells differ from progenitor cells in that they can grow indefinitely.

Particularly preferred cells suitable for myogenic cell therapy according to the invention, include, without limitation, myoblasts, satellite cells, mesoangioblasts, muscle-side population cells, cardiosphere-derived cells, and induced pluripotent stem cells (iPSC).

Myoblasts are myogenic precursor cells suitable for the present invention. Transplanted myoblasts are indeed able to fuse with the host fibers (Watt et al. Muscle Nerve. 1984; 7(9):741-50) and to increase voluntary muscle force (Karpati et al. Ann Neurol. 1993; 34(1):8-17).

Satellite cells (SC) are quiescent stem cells present in adult muscle, that are capable of undergoing muscle differentiation. Satellite cells can indeed give rise to regenerated muscle and establish a stem cell niche after forming a proper engraftment upon transplantation (Sacco et al. Nature. 2008; 456(7221):502-6).

Mesoangioblasts are vessel-associated progenitor cells resident in postnatal skeletal muscle that can differentiate into different mesodermal lineages, including muscle fibres (Dellavalle et al. Nat Commun. 2011; 2:499).

Muscle-side population (SP) cells are a population of myogenic progenitor cells found in muscle. Muscle SP cells can home to the skeletal muscle after systemic delivery, fuse with the host myofivers and even give rise to satellite cells upon transplantation (Biressi et al., J Clin Invest 2020; 130(11):5652-5664).

Cardiosphere-derived cells (CDCs), such as CAP-1002 (from Capricor Therapeutics), are heart progenitor cells that have regenerative, anti-inflammatory, anti-scarring and immune modulatory properties, and that have shown efficacy in treating DMD patients (Taylor et al., Neurology. 2019; 92(8): e866-e878).

At last, induced pluripotent stem cells (iPSC) can be differentiated into myogenic cells (Chal et al. Nat Biotechnol. 2015; 33(9):962-9), and even reconstitute the pool of satellite cells upon transplantation.

The skilled person would readily understand that any one of the (i) dynamin 2 inhibitors and (ii) therapies providing or restoring a functional dystrophin in a subject, as described herein, can be combined for the purpose of the present invention.

In a particularly preferred embodiment, (i) the dynamin 2 inhibitor is a nucleic acid molecule interfering specifically with dynamin 2 expression and (ii) the therapy providing the subject with a functional dystrophin is a dystrophin gene therapy.

More preferably, (i) the nucleic acid molecule interfering specifically with dynamin 2 expression is an antisense nucleic acid, and (ii) the functional dystrophin is a mini- or micro-dystrophin or a full-length dystrophin.

Even more preferably, (i) the antisense nucleic acid interfering specifically with dynamin 2 expression is complementary to at least one intron or the 3'UTR of Dynamin 2 pre-mRNA, preferably of at least intron 12 of dynamin 2 pre-mRNA, and (ii) the functional dystrophin is a mini- or micro-dystrophin.

Still preferably, (i) the antisense nucleic acid interfering specifically with dynamin 2 expression comprises or consisting of at least 8 contiguous nucleobases of any one of SEQ ID NO: 1 to 3135, and (ii) the functional dystrophin is a micro-dystrophin.

More preferably, (i) the antisense nucleic acid interfering specifically with dynamin 2 expression comprises the sequence SEQ ID NO: 2873 or SEQ ID NO: 3129, and (ii) the micro-dystrophin is the µDys5 micro-dystrophin or the ΔR4-R23/ΔCT micro-dystrophin.

Even more preferably, (i) the antisense nucleic acid interfering specifically with dynamin 2 expression comprises the sequence SEQ ID NO: 2873 or SEQ ID NO: 3129, and (ii) the micro-dystrophin is provided with an AAV vector as described above, such as an rAAV9-CK8-micro-dystrophin vector, an rAAVrh74-MHCK7-micro-dystrophin vector, or an rAAV9-Sp5.12-micro-dystrophin vector.

Yet, more preferably, (i) the antisense nucleic acid interfering specifically with dynamin 2 expression comprises the sequence SEQ ID NO: 2873 or SEQ ID NO: 3129, and (ii) the micro-dystrophin is provided with the rAAV9-CK8-µDys5 micro-dystrophin vector, the rAAVrh74-MHCK7-ΔR4-R23/ΔCT micro-dystrophin vector, or the rAAV9-Sp5.12-ΔR4-R23/ΔCT micro-dystrophin vector.

The combination therapy comprising a (i) dynamin 2 inhibitor and a (ii) therapy providing or restoring a functional dystrophin, as described herein, may be suitable for administration to a cell, tissue and/or an organ of individuals affected by or at risk of suffering from a dystrophin-related myopathy, and may be administered *in vivo, ex vivo* or *in vitro.* Since dystrophin-related myopathy affects muscles, it is preferred that said cells are muscle cells, that said tissue is a muscular tissue and/or that said organ comprises or consists of a muscular tissue.

Whether the combination therapy is administered simultaneously, separately or sequentially, each of therapy can be delivered as it is to the subject, or be formulated to be compatible with their intended route of administration.

For example, the combination therapy can be formulated in a combined pharmaceutical composition, or in separate pharmaceutical compositions, in a form suitable for parenteral, oral or topical administration, such as a liquid suspension, a solid dosage form (granules, pills, capsules or tablets), or a paste or gel.

For the purposes of the invention, a particularly preferred form of administration is parenteral administration, such as subcutaneous, intradermal, intravenous, or intramuscular administration. Intramuscular administration, in particular by injection, is herein preferred.

It is within the skill of the person in the art to formulate such pharmaceutical composition(s) in accordance with standard pharmaceutical practice (see, e.g., Remington: The Science and Practice of Pharmacy (20th ed.), ed. A. R. Gennaro, Lippincott Williams & Wilkins, 2000 and Encyclopedia of Pharmaceutical Technology, eds. J. Swarbrick and J. C. Boylan, 1988-1999, Marcel Dekker, New York). Pharmaceutical compositions according to the invention may notably be formulated to release the combination therapy immediately upon administration or at any predetermined time or time period after administration.

When formulated within a pharmaceutical composition, the combination therapy can be further combined with a pharmaceutically acceptable excipient.

As used herein, the term a *"pharmaceutically acceptable excipient"* means an inactive or inert, and therefore nontoxic, component, as it has no pharmacological action itself, which can be used to improve properties of a composition, such as shelf-life, retention time at the application site, consumer acceptance, etc. It includes, without limitation, surfactants (cationic, anionic, or neutral); surface stabilizers; other enhancers, such as preservatives, wetting or emulsifying agents; solvents; buffers; salt solutions; dispersion medium; isotonic and absorption delaying agents, and the like; that are physiologically compatible.

As explained above, the combination therapy is provided in a therapeutically effective amount so as to treat the dystrophin-related myopathy.

In a preferred embodiment, the therapeutically effective amount of dynamin 2 inhibitor is an amount sufficient to reduce the dynamin 2 expression, activity or function in a level equal or less than the normal level. The normal level of reference is the level of dynamin 2 expression, activity or function of subjects that do not suffer from a dystrophin-related myopathy.

In a preferred embodiment, the therapeutically effective amount of therapy providing or restoring a functional dystrophin is an amount sufficient to restore at least 1%, 5%, 10%, or 15%, preferably at least 10%, or 15%of expression, or function of dystrophin. The level of reference for dystrophin expression or function is the one of the subject prior to the treatment. In that regard, it should be further understood that the level of expression or function of dystrophin to be reached does not need to be achieved in all myofibers, but rather only in about 50% of myofibers (Sun et al., Genes 2020, 11, 837: 1-25).

Those of skill in the art will recognize that such parameters are normally worked out during clinical trials.

In another aspect, the present invention relates to (i) a dynamin 2 inhibitor and (ii) a therapy providing or restoring a functional dystrophin in a subject, as described above, as a combined preparation or therapy for simultaneous, separate or sequential use in the treatment of a dystrophin-related myopathy in said subject.

The invention relates to the simultaneous, separate or sequential use of (i) a dynamin 2 inhibitor and (ii) a therapy providing or restoring a functional dystrophin in a subject, as described above, for the preparation of a medicament for the treatment of a dystrophin-related myopathy in said subject.

Further provided is therefore a method for treating a dystrophin-related myopathy in a subject in need thereof, said method comprising the simultaneous, separate or sequential administration, to said subject, of a therapeutically effective amount of (i) a dynamin 2 inhibitor and (ii) a therapy providing or restoring a functional dystrophin in said subject.

Preferred embodiments, as described above, apply herein *mutatis mutandis.*

In another aspect, the present invention relates to a pharmaceutical composition comprising (i) a dynamin 2 inhibitor and (ii) a therapy providing or restoring a functional dystrophin in a subject, as described above, and optionally a pharmaceutically acceptable excipient.

Preferred embodiments, as described above, apply herein *mutatis mutandis.*

The present invention will be better understood in the light of the following detailed experiments. Nevertheless, the skilled artisan will appreciate that the present examples are not limitative and that various modifications, substitutions, omissions, and changes may be made without departing from the scope of the invention.

### EXAMPLES

### EXAMPLE 1

### 1. MATERIALS AND METHODS

### 1.1. Antisense oligonucleotides (ASO)

### ASO targeting Dmd

An antisense oligonucleotide targeting *Dmd* (M23D (+02-18)) was selected for their capacity to induce exon-skipping of exon 23 in the *Dmd* mouse gene, to remove the mutation in this exon and allow for expression of truncated dystrophin, as previously validated by Heemskerk al. (Mol Ther 2010, 18(6): 1210-1217), Cao et al. (Plos One 2014, 9(11):e111079), and Van Putten, et al. (Nucleic Acid Ther 2019, 29(2): 92-103. The ASO control sequence has no homology to the mouse genome (M23D sense oligo). These oligonucleotides were herein synthesized using an automated DNA synthesizer and chemically modified with a phosphorothioate in the backbone.

### ASO targeting Dnm2

The antisense oligonucleotide targeting *Dnm2* (ASO-Dnm2) was selected for its capacity to efficiently knockdown DNM2, as previously validated by Tasfaout et al. (Nat Commun 2017, 8: 15661) and Buono et al. (Proc Natl Acad Sci USA 2018, 115(43): 11066-11071). This ASO more specifically targets exon 17 of the *Dnm2* mouse gene. A random control ASO sequence not targeting any known mouse gene was used as a control (ASO-ctrl). These oligonucleotides were herein synthesized using an automated DNA synthesizer and chemically modified with phosphorothioate in the backbone and cEt modifications on the wings with a deoxy gap (3-10-3 design), as previously described (Tasfaout, et al. 2017, Nat Commun 8: 15661).

All ASOs used in the present study are described in Table 1 below. All ASOs were dissolved in filtered and autoclaved sterile D-PBS (Life Technologies, #14190-144).

**Table 1. Antisense oligonucleotides**

| **ASO name** | **ASO target** | **ASO sequence (5' to 3') (SEQ ID NO:)** | **ASO chemistry** |
|---|---|---|---|
| M23D (+02-18) | *Dmd* | GGCCAAACCUCGGCUUACCU (SEQ ID NO: 3157) | 2'O-methyl-phosphorothioate (2OMe) |
| M23D sense oligo (control) | | AGGUAAGCCGAGGUUGGCC (SEQ ID NO: 3158) | 2'O-methyl-phosphorothioate (2OMe) |
| ASO-ctrl (control) | *Dnm2* | GGCCAATACGCCGTCA (SEQ ID NO:3159) | Phosphorothiorate Constrained ethyl (cEt) |
| ASO-Dnm2 | | GGCATAAGGTCACGGA (SEQ ID NO: 3160) | Phosphorotiorate Constrained ethyl (cEt) |

### 1.2. Animal experiments

Animal experimentation ethics application #30975 was approved by MESR. 60 DBA/2J *mdx* (D2.B10-*Dmd^{mdx}*; referred to as *Dmd*^{*mdx*/*y*} mice) (Van Putten et al., FASEB J2019, 33(7): 8110-8124) and 15 DBA/2J wildtype mice (wt; referred to as *Dmd*^{+/*y*} mice) were purchased from the Jackson Laboratory. Mice were handled according to the French and European legislation on animal care and experimentation. Weekly injections of antisense oligonucleotides were performed in DBA/2J *mdx* and DBA/2J wt mice, from 8 to 19 weeks of age, as described in Table 2 below. Mice were then sacrificed 10 days after the final injection.

**Table 2. Study design. i.v. = intravenous (200mg/kg, once a week), i.p. = intraperitoneal (25mg/kg, once a week).**

| **Mouse genotypes (number tested)** | **ASO administration** |
|---|---|
| *Dmd*^{+/*y*} (wt) (x15) | M23D sense oligo (i.v.) + ASO-ctrl (i.p.) |
| *Dmd*^{*mdx*/*y*} (x15) | M23D sense oligo (i.v.) + ASO-ctrl (i.p.) |
| *Dmd*^{*mdx*/*y*} (x15) | M23D (+02-18) (i.v.) |
| *Dmd*^{*mdx*/*y*} (x15) | ASO-Dnm2 (i.p.) |
| *Dmd*^{*mdx*/*y*} (x15) | ASO ASO-Dnm2 (i.p.) + M23D (+02-18) (i.v.) |

### 1.3. Hanging test

Whole-body strength was evaluated at 19 weeks old using the 10-minute hanging test. Each mouse was placed in the middle of a grid and the grid was turned upside down above a cage with bedding. The suspending animal should hold on to the grid with all four limbs in order to avoid falling. The grid was placed at a height of approximately 40 cm above the bedding to make sure that the mouse was not influenced to jump. The latency time for the mouse to fall in the cage was measured, with a cut-off time of 10 minutes. Each animal was given a maximum of 3 trials per session, with 2 minutes minimum recovery period between trials. Mice that reach the fixed cut-off time of 10 minutes, independent of the trial number, were allowed to stop with the experiment, while others were retested for a maximum of three times. The maximum hanging time was recorded.

### 1.4. Forelimbs grip strength test

Grip strength was evaluated at 19 weeks old using a grip strength meter. The 2 front paws of the animal were placed on the meter grid, then the mouse was pulled backwards until it released its grip. A total of 5 trials per mouse, separated by around 10 sec, were performed. The same experimenter performed all tests. The three highest values were averaged and normalized to body weight.

### 1.5. PCR and sequencing analysis

For all mice, RNA was isolated from muscles (diaphragm and quadriceps) using MagMAX^{™} mirVana^{™} Total RNA Isolation Kit (Applied Biosystems) and KingFisher Flex System (ThermoFisher). Reverse transcription was carried out on a 250 ng aliquot using SuperScript^{™} IV Reverse Transcriptase (Invitrogen).

RT-PCR was performed with Phire Tissue Direct PCR Master Mix ^{™} (Thermo Scientific, F-170L), using the primers listed in Table 3 below. PCR clean-up was performed with NucleoSpin Gel and PCR Clean up kit (740609.50, Macherey-Nagel) and samples were sequenced.

**Table 3. Primers used for RT-PCR and sequencing analysis relating to Dmd**

| **Primer name** | **Primer sequence (5' to 3')** | **SEQ ID NO:** |
|---|---|---|
| mDMD_Exon20 Forward n2 | CCCAGTCTACCACCCTATCAGAGC | SEQ ID NO: 3161 |
| mDMD_Exon24 Reverse n2 | CCTGCCTTTAAGGCTTCCTT | SEQ ID NO: 3162 |
| mDMD_Exon20 Forward n1 | CAGAATTCTGCCAATTGCTGAG | SEQ ID NO: 3163 |
| mDMD_Exon26 Reverse n1 | TCTTCAGCTTGTGTCATCC | SEQ ID NO: 3164 |
| mDMD_Exon22 Forward n1 | GGAGGAGAGACTCGGGAAATTAC | SEQ ID NO: 3165 |
| mDMD_Exon22 Forward n2 | CCAGCAGTCAGAAAGCAAAC | SEQ ID NO: 3166 |
| mDMD_Exon24 Reverse | GGCAGGCCATTCCTCTTTCAG | SEQ ID NO: 3167 |

Quantitative RT-PCR (qPCR) was also performed with PowerUp^{™} SYBR^{™} Green Master Mix (Applied Biosystems) in a Quant Studio 3 Real-Time PCR System (Applied Biosystems), using the primers listed in Table 4 below. The relative expression of *Dnm2* mRNA was normalized to *Rpl27* and *Tbp.*

**Table 4. Primers used for qPCR analysis relating to Dnm2**

| **Gene** | **Forward primer (5' to 3') (SEQ ID NO:)** | **Reverse primer (5' to 3') (SEQ ID NO:)** |
|---|---|---|
| *Rpl27* | AAGCCGTCATCGTGAAGAACA (SEQ ID NO: 3168) | CTTGATCTTGGATCGCTTGGC (SEQ ID NO: 3169) |
| *Tbp* | ACCGTGAATCTTGGCTGTAAAC (SEQ ID NO: 3170) | GCAGCAAATCGCTTGGGATTA (SEQ ID NO: 3171) |
| *Dnm2* | ACCCCACACTTGCAGAAAAC (SEQ ID NO: 3172) | CGCTTCTCAAAGTCCACTCC (SEQ ID NO: 3173) |

### 1.6. Histological analysis of skeletal muscle

Air dried transverse cryosections (8 µm) of diaphragm muscle were fixed and stained with Sirius Red, and image acquisition performed with an Echo Rebel microscope (Echo Discovery, USA). The diaphragm thickness was calculated in FIJI analysis software (Rasband, W.S., ImageJ, U. S. National Institutes of Health, Bethesda, Maryland, USA, http://rsb.info.nih.gov/ij/, 1997-2009).

### 1.7. Statistics

Statistical analyses were performed with the software GraphPad Prism 9.1.0. The tests performed are indicated in Figure legends.

### 2. RESULTS

### 2.1. Validation of the antisense oligonucleotides' efficiency

### ASO targeting Dmd

The sequencing analysis from RNA extracted from diaphragm and quadriceps muscles confirmed that the targeting of the *Dmd* mouse gene by the ASO M23D (+02-18) successfully induced exon skipping of the mutated exon 23 in the *Dmd* mRNA in *Dmd*^{*mdx*/*y*} mice, while the control ASO M23D sense oligo did not (Tables 5 and 6).

**Table 5. Expected sequences with and without exon-skipping of the Dmd gene**

| | |
|---|---|
| (^{∗}: reverse complementary sequence, i.e. from 3' to 5') | |
| Sequence without exon skipping (exon 23-24) | Exon 24 / Exon 23 TATGTGATT/CTGAAATTTTCGAAGTTTATTCATATGTTCTTCTAGCTTTTGGCAGC^{∗} (corresponding to SEQ ID NO: 3174 from 5' to 3') |
| Sequence with exon skipping (exon 22-24) | Exon 24 / Exon 22 TATGTGATT/CTGTAATTTCCCGAGTCTCTCCTCCATTATTTCATATTCAGTAACAC^{∗} (corresponding to SEQ ID NO: 3175 from 5' to 3') |

**Table 6. Sequencing analysis following ASO administration to the mice**

| | |
|---|---|
| (^{∗}: reverse complementary sequence, i.e. from 3' to 5') | |

| **ASO administered** | **Sequencing result** |
|---|---|
| M23D sense oligo (Ctrl) | TATGTGATT/CTGAAATTTTCGAAGTT (...)^{∗} (corresponding to SEQ ID NO: 3174 from 5' to 3') |
| M23D (+02-18) | TATGTGATT/CTGTAATTTCCCGAGTCTC (...)^{∗} corresponding to (SEQ ID NO: 3175 from 5' to 3') |
| M23D (+02-18) and ASO-Dnm2 | TATGTGATT/CTGTAATTTCCCGAGTCTCTCCT (...)^{∗} (corresponding to SEQ ID NO: 3175 from 5' to 3') |

### ASO targeting Dnm2

The efficiency of the ASO targeting the *Dnm2* gene was previously validated by Tasfaout et al. (Nat Commun 2017, 8: 15661) and Buono et al. (Proc Natl Acad Sci USA 2018, 115(43): 11066-11071).

As shown in **Figure 1****,** we observed herein a reduction of *Dnm2* mRNA expression following ASO-Dnm2 administration in the diaphragm muscle **(A)** (by about 60%) and the quadriceps muscle **(B)** (by about 45%) in *Dmd*^{*mdx*/*y*} mice injected with ASO-Dnm2 alone or with the combination ASO-Dnm2 + M23D (+02-18), compared to *Dmd*^{+/*y*} mice injected with the control ASOs, ASO-ctrl + M23D sense oligo. The levels of *Dnm2* mRNA were similar to *Dmd*^{+/*y*} mice injected with the control ASOs in *Dmd*^{*mdx*/*y*} mice injected with the control ASOs or the exon-skipping ASO M23D (+02-18) in the diaphragm. In the quadriceps, levels of *Dnm2* mRNA were slightly elevated in *Dmd*^{*mdx*/*y*} mice injected with the control ASOs or the exon-skipping ASO M23D (+02-18). There was no effect of the ASO M23D (+02-18) on *Dnm2* mRNA expression, in either the diaphragm or quadriceps, as expected.

As shown in **Figure 2****,** at the protein level, reductions in *Dnm2* mRNA levels translated into reductions in DNM2 protein levels in *Dmd*^{*mdx*/*y*} mice injected with ASO-Dnm2 alone or with the combination ASO-Dnm2 + M23D (+02-18), in both the diaphragm **(A)** and quadriceps **(B)** muscles. Indeed, in *Dmd*^{*mdx*/*y*} mice injected with the control ASOs or the exon-skipping ASO M23D (+02-18), DNM2 protein levels were elevated compared to *Dmd*^{+/*y*} mice injected with the control ASOs, in the diaphragm (by about 95-100%) and the quadriceps (by about 40-75%). These results also confirmed that the exon-skipping ASO had no effect on DNM2 expression. By contrast, the ASO-Dnm2 reduced DNM2 protein levels in *Dmd*^{*mdx*/*y*} mice by 30% in the diaphragm and by 15% in the quadriceps, while the combination of ASO-Dnm2 + M23D (+02-18) reduced DNM2 protein levels by 45% in the diaphragm and by 25% in the quadriceps, compared to *Dmd*^{*mdx*/*y*} mice injected with control ASOs.

These results suggest that the combination of reducing DNM2 expression and restoring some dystrophin expression has a synergistic effect on DNM2 protein level reduction.

### 2.2. Hanging test

Whole-body strength was investigated using the 10-minute hanging test at 19 weeks of age. Indeed, *Dmd*^{*mdx*/*y*} mice on the DBA/2J background were previously shown to perform significantly worse on this test compared to *Dmd*^{+/*y*} wild type control mice (Van Putten, et al. FASEBJ2019, 33(7): 8110-8124).

As expected and shown in **Figure 3****,** *Dmd*^{*mdx*/*y*} mice treated with control ASOs were not able to hang for the 10 minutes duration of the test, with a mean hanging time of 279.5 s (median hanging time of 223 s), and 0% of the mice able to hold for 10 minutes. Individual treatments with either M23D (+02-18) or ASO-Dnm2 similarly improved, albeit not significantly, the ability of *Dmd*^{*mdx*/*y*} mice to hang, with mean hanging times of 387.9 s and 398.7 s, respectively (median holding times of 395.5 s and 372 s, respectively), and 42% and 40% of the mice being able to hold for 10 minutes, respectively. Surprisingly, the combination therapy of ASO-Dnm2 and M23D (+02-18) provided a significantly greater improvement in whole-body strength than either treatment alone, with a mean hanging time of 478.7 s (median holding time of 600 s) and 53% of the mice able to hang for 10 minutes.

These results demonstrate that both restoring some truncated dystrophin expression or reducing *Dnm2* expression have a therapeutic effect on whole-body strength, and, more importantly, that this effect is synergistic when both therapies are combined.

### 2.3. Grip strength test

Muscle force was investigated using the forelimbs grip strength test at 19 weeks of age. Indeed, *Dmd*^{*mdx*/*y*} mice on the DBA/2J background were previously shown to perform significantly worse on this test compared to *Dmd*^{+/*y*} wild type control mice (Van Putten, et al. FASEB J2019, 33(7): 8110-8124).

As shown in **Figure 4****,** *Dmd*^{*mdx*/*y*} mice treated with control ASOs had a mean muscle force normalized to body weight (BW) of 3.35 g/gBW. Individual treatments with either M23D (+02-18) or ASO-Dnm2 improved, albeit not significantly, the muscle strength of *Dmd*^{*mdx*/*y*} mice, with a mean of 3.81 g/gBW and 3.64 g/gBW, corresponding to 13.8% and 8.8% increase in strength compared to the control, respectively. Surprisingly, the combination therapy of ASO-Dnm2 and M23D (+02-18) provided a significantly greater improvement in muscle strength than either treatment alone, with a mean of 3.98 g/gBW, corresponding to 18.9% increase in strength compared to the control.

These results demonstrate that the combination of *Dnm2* expression reduction with restoration of truncated dystrophin expression has a synergistic therapeutic effect on muscle force.

### 2.4. Histological analysis of skeletal muscle

Diaphragm thickness of treated mice was analyzed on histology sections stained with Sirius Red. Images of histological sections are shown in **Figure 5****,** and **Figure 6** illustrates the measures of diaphragm thickness (µm).

In *Dmd*^{*mdx*/*y*} mice treated with control ASOs, the diaphragm was enlarged compared to *Dmd*^{+/*y*} wild type control mice. Injections of the exon-skipping ASO M23D (+02-18) in *Dmd*^{*mdx*/*y*} mice did not reduce diaphragm thickness. By contrast, injections of ASO-Dnm2 in *Dmd*^{*mdx*/*y*} mice reduced diaphragm thickness, while the combination therapy of ASO-Dnm2 + M23D (+02-18) provided a superior amelioration than ASO-Dnm2 alone, showing a synergistic effect of this combination therapy.

### EXAMPLE 2

An antisense oligonucleotide targeting *Dnm2* as shown in **Figure 6** is co-administered with the viral vector rAAVrh74-MHCK7-ΔR4-R23/ΔCT micro-dystrophin in patients suffering from DMD or BMD.

## Claims

1. A combination therapy for use in the treatment of a dystrophin-related myopathy in a subject in need thereof, the combination therapy comprising (i) a dynamin 2 inhibitor and (ii) a therapy providing or restoring a functional dystrophin in the subject.

2. The combination therapy for use according to claim 1, wherein the dystrophin-related myopathy is Duchenne muscular dystrophy or Becker muscular dystrophy, preferably Duchenne muscular dystrophy.

3. The combination therapy for use according to claim 1 or 2, wherein the dynamin 2 inhibitor is selected from the group consisting of a nucleic acid molecule interfering specifically with dynamin 2 expression, an antibody directed against dynamin 2, a genome editing system comprising a nuclease engineered to target the *DNM2* gene, and a small molecule inhibiting the dynamin 2 activity, expression or function, and any combinations thereof.

4. The combination therapy for use according to claim 3, wherein the nucleic acid molecule interfering specifically with dynamin 2 expression is an antisense nucleic acid, an RNAi nucleic acid, or a ribozyme interfering specifically with dynamin 2 expression.

5. The combination therapy for use according to claim 3 or 4, wherein the nucleic acid molecule interfering specifically with dynamin 2 expression is complementary to at least one intron or the 3'UTR of dynamin 2 pre-mRNA, preferably to at least intron 12, intron 13, intron 11, intron 1, intron 14 of dynamin 2 pre-mRNA, more preferably is an antisense nucleic acid comprising or consisting of at least 8 contiguous nucleobases of any one of SEQ ID NO: 1 to 3135.

6. The combination therapy for use according to claim 3 or 4, wherein the nucleic acid molecule interfering specifically with dynamin 2 expression induces exon-skipping within a dynamin 2 pre-mRNA, preferably of at least exon 2 and/or 8 of dynamin 2 pre-mRNA, more preferably is an antisense nucleic acid comprising or consisting of the sequence SEQ ID NO: 3136 or SEQ ID NO: 3137.

7. The combination therapy for use according to claim 3 or 4, wherein the nucleic acid molecule interfering specifically with dynamin 2 expression is complementary to at least one exon of dynamin 2 mRNA, preferably to at least exon 1, 4, 5, 12b, 13, 15, 17 and/or 21 of dynamin 2 mRNA, more preferably is an RNAi nucleic acid comprising or consisting of any one of the sequences SEQ ID NO: 3138 to SEQ ID NO: 3151.

8. The combination therapy for use according to any one of the preceding claims, wherein the therapy providing or restoring a functional dystrophin in the subject is selected from the group consisting of: dystrophin gene therapy, exon-skipping therapy, codon-read-through therapy, genome editing therapy, myogenic cell therapy, and any combinations thereof.

9. The combination therapy for use according to claim 8, wherein the dystrophin gene therapy provides a functional dystrophin selected from a mini-dystrophin, a micro-dystrophin or a full-length dystrophin, preferably selected from the micro-dystrophins ΔDysM3, ΔDysH1, ΔDysH4, ΔDysHAH3, ΔDysAX2, ΔDysAX11, Δ3447, Δ3510, Δ3531, Δ3849, Δ3990, Δ4173, ΔR1-R24, ΔR4-23/ΔCT, ΔR4-R23, ΔR2-R21,ΔR2-R21+H3, Δ3788, ABS1µDys, ABS1.2µDys, ΔR2-15/ΔR18-23/ΔC, ΔR3-15/ΔR18-23/ΔC, ΔR3-15/ΔR17-23/ΔC, M1, M2, M3, M4, ΔH2-R24/ΔCT, ΔH2-R23+H3/ΔCT, ΔH2+R23+R18+H3/ΔCT, ΔR4-R23/ΔCT/Δ, MD1, MD2, ΔCS2, R6-17/H3/ΔC, or mDys5; or the mini-dystrophin Δ17-48, or any combinations thereof.

10. The combination therapy for use according to claim 8, wherein the exon-skipping therapy induces exon-skipping of at least one mutated exon of a dystrophin pre-mRNA, preferably of at least one of exon 51, 53, 45, 44, 52, 43, 23, 35, 50, 52, 7, 8, 17 and/or 55 of a dystrophin pre-mRNA, more preferably comprises an exon-skipping antisense nucleic acid such as eteplirsen, drisapersen, SRP50-51, suvodirsen, golodirsen, viltolarsen, WVE-5N31, casimersen, DS-5141b, NS-89/NCNP-02, or any combinations thereof.

11. The combination therapy for use according to claim 8, wherein the codon-read-through therapy comprises a small molecule suppressing premature termination codons in a dystrophin mRNA, said small molecule being preferably selected from the group consisting of aminoglycoside compounds such as geneticin, gentamycin, gentamycin B1, paromomycin, amikacin, NB30, NB54, NB74, NB84, NB124, TC007; and non-aminoglycoside compounds such as ataluren, PCT-414, RTC13, RTC14, GJ071, GJ072, RTC204, RTC219, BZ6, BZ16, negamycin, 3-epideoxynegamycin, leucyl-3-epi-deoxynegamycin, TCP-112, TCP-182, TCP-1109, amlexanox, clitocine, 2,6-diaminopurine, tylosin, josamycin, spiramycin, erythromycin, azithromycin; functional analogs thereof, and any combinations thereof.

12. The combination therapy for use according to claim 8, wherein the myogenic cellular therapy comprises precursor cells, progenitor cells, or stem cells that are capable of displaying a myogenic phenotype, preferably comprises myoblasts, satellite cells, mesoangioblasts, muscle-side population cells, cardiosphere-derived cells, induced pluripotent stem cells (iPSC), or any combinations thereof.

13. The combination therapy for use according to any one of the preceding claims, wherein:
• the dynamin 2 inhibitor is administered in an amount sufficient to reduce the dynamin 2 expression, activity, expression or function in a level equal or preferably less than the normal level; and/or
• the therapy providing or restoring a functional dystrophin is administered in an amount sufficient to restore at least 1%, 5%, 10% or 15% of expression or function of dystrophin.

14. (i) A dynamin 2 inhibitor and (ii) a therapy providing or restoring a functional dystrophin, as defined in any one of the preceding claims, as a combined preparation for simultaneous, separate or sequential use in the treatment of a dystrophin-related myopathy, such as Duchenne muscular dystrophy or Becker muscular dystrophy.

15. A pharmaceutical composition comprising (i) a dynamin 2 inhibitor and (ii) a therapy providing or restoring a functional dystrophin, as defined in any one of the preceding claims, and optionally a pharmaceutically acceptable excipient.
